(19) Europäisches Patentamt
European Patent Office
Office européen des brevets

(11) **EP 4 725 391 A1**

(12) **EUROPEAN PATENT APPLICATION**
published in accordance with Art. 153(4) EPC

(43) Date of publication:
**15.04.2026 Bulletin 2026/16**

(21) Application number: **23940851.1**

(22) Date of filing: **01.11.2023**

(51) International Patent Classification (IPC):
$A61B\ 3/00^{(2006.01)}$    $A61B\ 3/12^{(2006.01)}$
$A61B\ 3/14^{(2006.01)}$    $G16H\ 50/20^{(2018.01)}$
$G16H\ 30/20^{(2018.01)}$

(52) Cooperative Patent Classification (CPC):
**A61B 3/00; A61B 3/12; A61B 3/14; G16H 30/20; G16H 50/20**

(86) International application number:
**PCT/KR2023/017322**

(87) International publication number:
**WO 2024/253266 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.06.2023 KR 20230074433**

(71) Applicant: **Mediwhale Inc.**
**Seoul 06049 (KR)**

(72) Inventors:
• **CHOI, Tae Geun**
**Seoul, 02207 (KR)**
• **LEE, Geun Yeong**
**Seoul 08807 (KR)**
• **RIM, Hyung Taek**
**Seoul 06601 (KR)**
• **PARK, Jungtak**
**Seoul 03722 (KR)**
• **JOO, Young Su**
**Yongin-si, Gyeonggi-do, 16995 (KR)**

(74) Representative: **Westphal, Mussgnug & Partner Patentanwälte mbB**
**Am Riettor 5**
**78048 Villingen-Schwenningen (DE)**

(54) **METHOD AND DEVICE FOR DIAGNOSING KIDNEY DISEASE**

(57) A method and device for diagnosing renal disease are disclosed. A control method of a diagnostic device according to one embodiment comprises: obtaining a retinal image of a subject; and obtaining renal disease diagnostic information regarding the subject using a machine learning model based on the retinal image, wherein the machine learning model includes a first model and a second model, wherein the first model is a neural network model, and wherein the second model is a regression-based machine learning model.

FIG.19

**EP 4 725 391 A1**

**Description**

[Technical Field]

The present invention relates to a method and device for diagnosing renal disease.

[Background Art]

Retinal examination can observe abnormalities in the retina, optic nerve, and macula, and results can be confirmed relatively simply through imaging, making it diagnostic data frequently utilized in ophthalmology. Meanwhile, due to the recent leap forward in artificial intelligence technology, the development of diagnostic artificial intelligence is being actively conducted in the field of medical diagnosis, particularly in image-based diagnosis fields. Global companies are also sparing no investment in the development of artificial intelligence for analyzing various medical imaging data, such as by inputting large-scale data through collaboration with the medical community, and some companies have succeeded in developing artificial intelligence diagnostic tools that output excellent diagnostic results.
Since blood vessels in the body can be observed non-invasively using retinal images, expansion of diagnostic application using retinal images is being requested not only for ophthalmic diseases but also in relation to renal disease.

[Disclosure]

[Technical Problem]

The technical problem of the present application is to provide a method for diagnosing renal disease that obtains information regarding renal disease with high accuracy using retinal images and a machine learning model.
The technical problem of the present application is not limited to the above-mentioned problem, and problems not mentioned will be clearly understood by those having ordinary knowledge in the technical field to which the present application belongs from the present specification and the attached drawings.

[Technical Solution]

According to one aspect, a control method of a diagnostic device according to one embodiment comprises: obtaining a retinal image of a subject; and obtaining renal disease diagnostic information regarding the subject using a machine learning model based on the retinal image, wherein the machine learning model includes a first model and a second model, wherein the first model is a neural network model, and wherein the second model may be a regression-based machine learning model.
The technical solution is not limited to the above-mentioned solutions, and technical solutions not mentioned will be clearly understood by those having ordinary knowledge in the technical field to which the present application belongs from the present specification and the attached drawings.

[Advantageous Effects]

According to the present application, information regarding renal disease can be obtained with high accuracy using retinal images and a machine learning model.
The effects of the invention of the present application are not limited to the above-mentioned effects, and effects not mentioned will be clearly understood by those having ordinary knowledge in the technical field to which the present application belongs from the present specification and the attached drawings.

[Description of Drawings]

FIG. 1 illustrates a diagnostic system according to one embodiment.

FIG. 2 is a block diagram for explaining a learning device according to one embodiment.

FIG. 3 is a block diagram for explaining a diagnostic device according to one embodiment.

FIG. 4 illustrates a diagnostic system according to one embodiment.

FIG. 5 is a block diagram for explaining a client device according to one embodiment of the present invention.

FIG. 6 is a diagram for explaining a diagnostic process according to one embodiment of the present invention.

FIG. 7 is a diagram for explaining the configuration of a learning unit according to one embodiment of the present invention.

FIG. 8 is a conceptual diagram for explaining an image data set according to one embodiment of the present invention.

FIG. 9 is a block diagram for explaining a learning process of a diagnostic model according to one embodiment of the present invention.

FIG. 10 is a diagram for explaining the configuration of a diagnostic unit according to one embodiment of the present invention.

FIG. 11 is a diagram for explaining a diagnostic process according to one embodiment of the present invention.

FIG. 12 is a block diagram for explaining a diagnostic unit according to one embodiment of the present invention.

FIG. 13 is a diagram for explaining a diagnostic process according to one embodiment of the present invention.

FIG. 14 is a diagram for explaining a diagnostic system according to one embodiment of the present invention.

FIG. 15 is a diagram for explaining a serial diagnostic model according to one embodiment.

FIG. 16 is a diagram for explaining a serial diagnostic model according to another embodiment.

FIG. 17 is a diagram for explaining a serial diagnostic model according to yet another embodiment.

FIG. 18 is a diagram for explaining a diagnostic method using a diagnostic model according to one embodiment.

FIG. 19 is a diagram for explaining a method for diagnosing renal disease according to one embodiment.

FIG. 20 illustrates a diagnostic model for obtaining renal disease diagnostic information according to one embodiment.

FIG. 21 shows clinical characteristics of subjects according to Example 1.

FIGS. 22A and 22B are diagrams for explaining the incidence rate of renal disease-related events according to renal disease diagnostic information according to Example 1.

FIG. 23 is a diagram for explaining the performance of predicting the occurrence of renal disease-related events within 5 years of renal disease diagnostic information according to Example 1.

FIG. 24 is a diagram for explaining a method for diagnosing renal disease according to another embodiment.

FIG. 25 is a diagram for explaining a method for providing guide information regarding renal disease diagnostic information according to one embodiment.

FIG. 26 is a diagram for explaining a method for providing guide information using existing prescription information and renal disease diagnostic information according to one embodiment.

FIG. 27 is a diagram for explaining a method for predicting the progression rate of renal disease using renal disease diagnostic information according to one embodiment.

FIG. 28 is a diagram for explaining clinical characteristics of subjects according to Example 2.

FIGS. 29A and 29B are diagrams for explaining the incidence rate of renal disease-related events according to KDIGO grade and renal disease diagnostic information according to Example 2.

[Best Mode]

A control method of a diagnostic device according to one embodiment comprises: obtaining a retinal image of a subject; and obtaining renal disease diagnostic information regarding the subject using a machine learning model based on the retinal image, wherein the machine learning model includes a first model and a second model, wherein the first model is a neural network model, and wherein the second model may be a regression-based machine learning model.

[Mode for Invention]

The above-mentioned objects, features, and advantages of the present invention will become more apparent through the following detailed description related to the attached drawings. However, since the present invention can have various changes and can have various embodiments, specific embodiments will be illustrated in the drawings and described in detail below.
In the drawings, the thicknesses of layers and regions are exaggerated for clarity, and also, when an element or layer is referred to as being "on" or "above" another element or layer, it includes not only the case where it is directly on the other element or layer but also the case where another layer or another element is interposed therebetween. Throughout the specification, the same reference numerals indicate, in principle, the same elements. In addition, elements having the same function within the scope of the same idea appearing in the drawings of each embodiment are described using the same reference numerals.
When it is determined that a detailed description of a known function or configuration related to the present invention may unnecessarily obscure the gist of the present invention, the detailed description thereof will be omitted. In addition, numerals (for example, first, second, etc.) used in the description process of the present specification are merely identification symbols for distinguishing one element from another element.
In addition, the suffixes "module" and "unit" for elements used in the following description are given or used interchangeably only in consideration of ease of writing the specification, and do not have meanings or roles that are distinguished from each other per se.
The method according to the embodiment may be implemented in the form of program instructions that can be executed through various computer means and recorded on a computer-readable medium. The computer-readable medium may include program instructions, data files, data structures, etc., alone or in combination. The program instructions recorded on the medium may be specially designed and configured for the embodiment or may be known and available to those skilled in computer software. Examples of computer-readable recording media include magnetic media such as hard disks, floppy disks, and magnetic tapes, optical media such as CD-ROMs and DVDs, magneto-optical media such as floptical disks, and hardware devices specially configured to store and execute program instructions such as ROM, RAM, flash memory, etc. Examples of program instructions include not only machine language code such as that created by a compiler but also high-level language code that can be executed by a computer using an interpreter, etc. The above-described hardware devices may be configured to operate as one or more software modules to perform the operations of the embodiments, and vice versa.

1. Diagnosis Using Retinal Images

1.1. Diagnostic System and Process

1.1.1. Purpose and Definitions

Hereinafter, a diagnostic system and method for assisting medical staff's judgment regarding the presence or absence of disease or abnormality that serves as a basis for such judgment based on ocular images will be described. In the present specification, the term diagnosis may mean diagnostic assistance for assisting in the diagnosis of disease rather than directly diagnosing disease. Hereinafter, the term diagnosis is used for convenience of description, but this may mean diagnostic assistance.
In particular, a diagnostic method for constructing a machine learning model for diagnosing disease using deep learning techniques and assisting in the detection of the presence or absence of disease or abnormal findings using the constructed model will be described. In the present specification, an ocular image is an image including the eye of a subject, and may include various images such as a retinal image and/or a fundus image. In the present specification, the technology of the present specification is described focusing on retinal images for convenience of description, but is not limited thereto, and it goes without saying that the description of the present specification can also be applied to other ocular images.

The machine learning model described in the present specification may be designed based on various machine learning libraries. For example, the machine learning model may mean various types of models designed based on supervised, unsupervised, semi-supervised, or reinforcement learning artificial intelligence algorithms such as decision tree, random forest algorithm, stochastic gradient descent algorithm, neural network algorithm, k-nearest neighbor algorithm, linear regression, logistic regression, Cox proportional hazards model (survival model) (regression-based), support vector machine, k-means, Hierarchical Cluster Analysis (HCA), Expectation Maximization, Principal Component Analysis (PCA), Kernel PCA, Locally-Linear Embedding (LLE), t-distributed Stochastic Neighbor Embedding (t-SNE), Apriori, and Eclat.

Hereinafter, unless otherwise specifically described, the machine learning model is mainly described as a neural network model for convenience, but this does not necessarily mean only a model form based on a neural network algorithm, and it is obvious that it can be replaced with a model based on another algorithm within the functional and purpose scope of the invention described in the present specification.

According to one embodiment of the present invention, a diagnostic system or method for assisting in the diagnosis of at least one of ophthalmic disease, cardiovascular disease (and/or cardio-cerebrovascular disease), renal disease, and other systemic diseases based on retinal images may be provided.

Illustratively, in the present specification, ophthalmic disease may include at least one of Cataract, Glaucoma, Macular Degeneration, Diabetic Retinopathy, Epiretinal membrane, macular hole, high/degenerative myopia, melanoma, Retinal Detachment, Dry Eye Syndrome, Presbyopia, and Astigmatism.

In addition, cardiovascular disease may include at least one of Coronary Artery Disease (CAD), aortic valve stenosis, Hypertension, Arterial hypertension, Heart Failure, arrhythmia, Atrial Fibrillation, Valvular Heart Disease, Cardiomyopathy, Peripheral vascular disease, Peripheral Artery Disease (PAD), Heart Attack, Aneurysm, aortic aneurysm (for example, abdominal aortic aneurysm, thoracic aortic aneurysm), thrombotic disease (for example, deep vein thrombosis, pulmonary embolism), myocardial infarction, and cardio-cerebrovascular disease. In addition, cardio-cerebrovascular disease may include at least one of Stroke, ischemic stroke, cerebral infarction, cerebral hemorrhage, subarachnoid hemorrhage, transient ischemic attack, and death due to cardiovascular disease. Cardiovascular disease may include complications. In addition, complications may include aspiration pneumonia, dysphagia, decreased motor function, decreased language function, decreased cognitive function, sleep disorder, emotional disorder, cranial neuropathic pain, urinary tract infection, malnutrition, deep vein thrombosis, pressure ulcer, fall, pain, seizure, depression, etc.

In addition, renal disease may include at least one of Chronic Kidney Disease (CKD), Acute Kidney Injury (AKI), Kidney Stones, Nephrotic Syndrome, Glomerulonephritis, Polycystic Kidney Disease (PKD), Kidney Cancer, and Pyelonephritis. In addition, renal disease may include complications. In addition, complications may include side effects due to dialysis, hypotension, muscle cramps, nausea and vomiting, headache, dialysis disequilibrium syndrome, pruritus, arrhythmia, etc.

In addition, other systemic diseases may include at least one of diabetes, hypertension, hypotension, Alzheimer's disease, cytomegalovirus, and arteriosclerosis.

In addition, according to another embodiment of the present invention, various parameters of a subject can be predicted based on retinal images. For example, the parameters may include at least one of parameters representing physical information of the subject such as biological age, sex, height, weight, BMI index, body mass, body fat percentage, body muscle mass, etc. In addition, the parameters may include at least one of diagnostic value parameters such as hematocrit level, red blood cell count, white blood cell count, hemoglobin level, platelet count, total iron binding capacity (TIBC), iron level, ferritin (storage iron protein) level, total protein level, albumin level, aspartate transaminase level (AST), amino transaminase level, $\gamma$-GTP, $\gamma$-GT, alkaline phosphatase (ALP) level, globulin level, hepatitis antigen level, hepatitis antibody level, glycated hemoglobin level (HbA1C), blood urea nitrogen (BUN) level, creatinine level, uric acid level, total cholesterol level, high-density lipoprotein (HDL Cholesterol) level, low-density lipoprotein (LDL Cholesterol) level, triglyceride (TG) level, bicarbonate level, systolic blood pressure (SBP), diastolic blood pressure (DBP), etc.

According to yet another embodiment of the present invention, a diagnostic system or method for detecting abnormal retinal findings that can be used in the diagnosis of ophthalmic diseases or other diseases may be provided. For example, a diagnostic system or method for obtaining finding information such as color abnormality of the entire retina, Opacity, abnormality of cup to disc ratio (C/D ratio) of the optic disc, macular abnormality (for example, macular hole), abnormality of vessel diameter, course, etc., retinal artery diameter abnormality, retinal hemorrhage, microaneurysm, hard exudate, Epiretinal membrane, myelinated nerve fiber, chorioretinal atrophy, RNFL defect, occurrence of exudate, drusen, cataract, glaucoma, diabetic retinopathy, Tessellated fundus, Large optic cup, retinal vein occlusion (RVO), branch retinal vein occlusion (BRVO), central retinal vein occlusion (CRVO), retinal artery occlusion (RAO), Rhegmatogenous RD, Posterior serous/exudative RD, central serous chorioretinopathy (CSCR), VKH disease, Maculopathy, epiretinal membrane (ERM), Macular Hole (MH), Pathological myopia, Optic nerve degeneration, Optic atrophy, Severe hypertensive retinopathy, Disc swelling and elevation, Dragged disc, Pigmentary degeneration, Congenital disc abnormality, Retinitis pigmentosa, Bietti crystalline dystrophy, Peripheral retinal degeneration and break, Myelinated nerve fiber, Vitreous particles, Fundus neoplasm, Massive hard exudates, Yellow-white spots/flecks, Cotton-wool spots, Vessel tortuosity, Chorioretinal atrophy/coloboma, Preretinal haemorrhage, Fibrosis, Laser spots, Silicon oil in eye, Blur fundus, Blur fundus without PDR, Blur fundus with suspected PDR, etc., may be provided.

In the present specification, diagnostic information may be understood as encompassing diagnostic information according to the determination of the presence or absence of disease or finding information that serves as the basis thereof.

### 1.1.2. Diagnostic System Configuration

According to one embodiment of the present invention, a diagnostic system may be provided.

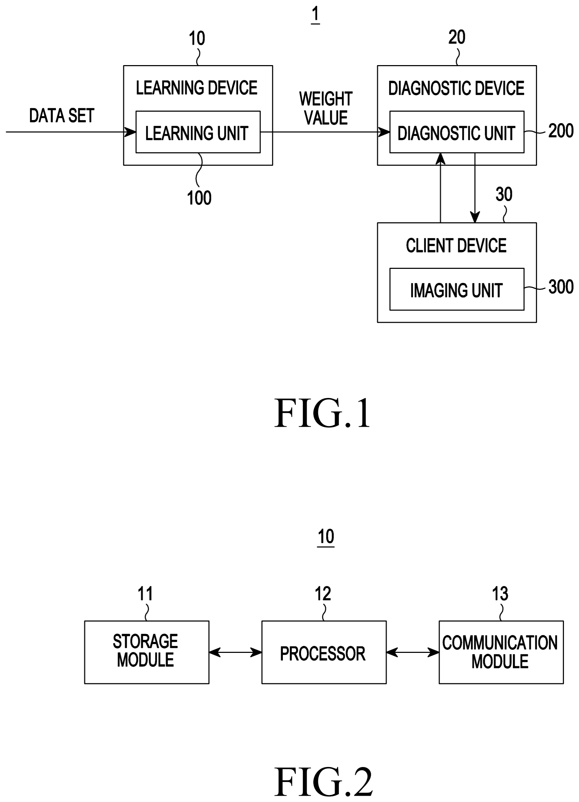

FIG. 1 illustrates a diagnostic system according to one embodiment of the present invention. Referring to FIG. 1, the diagnostic system (1) may include a learning device (10) that trains a diagnostic model, a diagnostic device (20) that performs diagnosis using the diagnostic model, and a client device (30) that obtains a diagnosis request. The diagnostic system (1) may include a plurality of learning devices, a plurality of diagnostic devices, or a plurality of client devices.

The learning device (10) may include a learning unit (100). The learning unit (100) may perform training of the diagnostic model. As an example, the learning unit (100) may obtain a retinal image data set and perform training of a diagnostic model that detects disease or abnormal findings from retinal images. The learning unit (100) is included in a processor of the learning device (10) to be described later, and may mean that the processing for learning of the processor is functionally represented.

The diagnostic device (20) may include a diagnostic unit (200). The diagnostic unit (200) may perform diagnosis of disease or acquisition of assitant information used for diagnosis using the diagnostic model. As an example, the diagnostic unit (200) may perform acquisition of diagnostic information using the diagnostic model trained by the learning unit. The diagnostic unit (200) is included in a processor of the diagnostic device (20) to be described later, and may mean that the processing for diagnosis of the processor is functionally represented.

The client device (30) may include an imaging unit (300). The imaging unit (300) may image a retinal image. The client device may be an ophthalmic retinal imaging device. Alternatively, the client device (30) may be a handheld device such as a smartphone or a tablet PC.

In the diagnostic system (1) according to the present embodiment, the learning device (10) obtains a data set and performs learning of the diagnostic model to determine the diagnostic model for use in diagnosis, the diagnostic device obtains diagnostic information according to a diagnostic target image using the determined diagnostic model when an information request is obtained from a client, and the client device may request information from the diagnostic device and obtain diagnostic information transmitted in response thereto.

A diagnostic system according to another embodiment may include a diagnostic device that performs learning of a diagnostic model and diagnosis using the same, and a client device. A diagnostic system according to yet another embodiment may include a diagnostic device that performs learning of a diagnostic model, acquisition of a diagnosis request, and diagnosis. A diagnostic system according to yet another embodiment may include a learning device that performs learning of a diagnostic model, and a diagnostic device that obtains a diagnosis request and performs diagnosis.

The diagnostic system disclosed in the present specification is not limited to the embodiments described above, and may be implemented in any form including a learning unit that performs learning of a model, a diagnostic unit that obtains diagnostic information according to the learned model, and an imaging unit that obtains a diagnostic target image.

Hereinafter, some embodiments of each device constituting the system will be described.

### 1.1.2.1. Learning Device

A learning device according to one embodiment of the present invention may perform training of a diagnostic model that assists diagnosis.

FIG. 2 is a block diagram for explaining a learning device according to one embodiment of the present invention. Referring to FIG. 2, the learning device (10) may include a processor (12) and a storage module (11).

The learning device (10) may include a processor (12). The processor (12) may control the operation of the learning device (10).

The processor (12) may include one or more of a Central Processing Unit (CPU), Random Access Memory (RAM), Graphic Processing Unit (GPU), one or more microprocessors, and other electronic components capable of processing input data according to predetermined logic. In addition, the processor (12) may be at least one or more.

The processor (12) may read system programs and various processing programs stored in the storage module (11). As an example, the processor (12) may develop processes, methods, etc. for performing diagnosis to be described later on RAM, and perform various processing according to the developed program. The processor (12) may perform learning of a diagnostic model to be described later.

The learning device (10) may include a storage module (11). The storage module (11) may store data and learning models necessary for learning.

The storage module (11) may be implemented as a non-volatile semiconductor memory, hard disk, flash memory, RAM, Read Only Memory (ROM), Electrically Erasable Programmable Read-Only Memory (EEPROM), or other tangible non-volatile recording media.

The storage module (11) may store various processing programs, parameters for performing processing of the programs, or data of such processing results. As an example, the storage module (11) may store a data processing process program for performing diagnosis to be described later, a diagnostic process program, parameters for performing each program, and data obtained by performing such programs (for example, processed data or diagnostic result values). In addition, the storage module (11) may store various diagnostic models to be described later.

The learning device (10) may include a separate learning unit. The learning unit may perform learning of the diagnostic model.

The learning unit may be included in the processor (12) described above. The learning unit may be stored in the storage module (11) described above. The learning unit may be implemented by some components of the processor (12) and the storage module (11) described above. For example, the learning unit may be stored in the storage module (11) and driven by the processor (12).

The learning device (10) may further include a communication module (13). The communication module (13) may perform communication with an external device. For example, the communication module (13) may communicate with a diagnostic device, server device, or client device to be described later. The communication module (13) may perform wired or wireless communication. The communication module (13) may perform bi-directional or unidirectional communication.

### 1.1.2.2. Diagnostic Device

The diagnostic device may obtain diagnostic information using a diagnostic model.

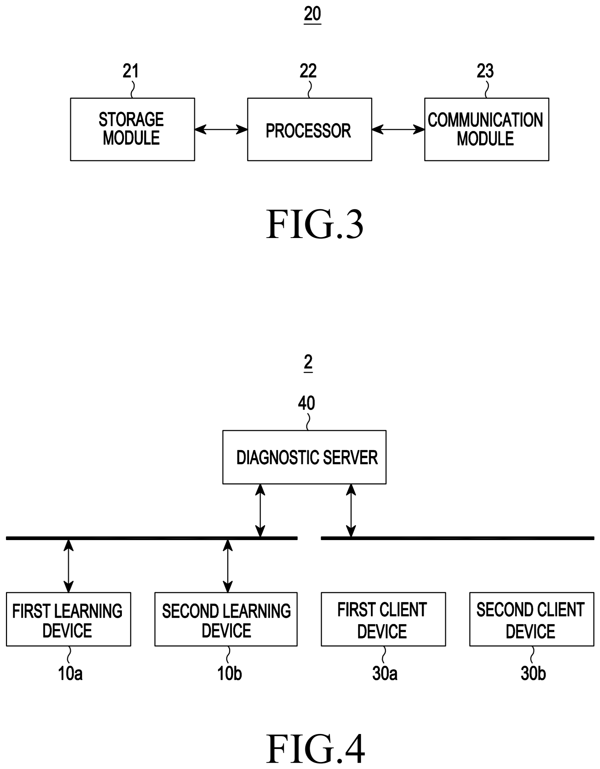

FIG. 3 is a block diagram for explaining a diagnostic device according to one embodiment of the present invention. Referring to FIG. 3, the diagnostic device (20) may include a processor (22) and a storage module (21).

The processor (22) may include one or more of a Central Processing Unit (CPU), Random Access Memory (RAM), Graphic Processing Unit (GPU), one or more microprocessors, and other electronic components capable of processing input data according to predetermined logic. In addition, the processor (22) may be at least one or more.

The processor (22) may read system programs and various processing programs stored in the storage module (21). As an example, the processor (22) may develop processes, methods, etc. for performing diagnosis to be described later on RAM, and perform various processing according to the developed program. The processor (22) may generate diagnostic information using a diagnostic model. The processor (22) may obtain diagnostic data for diagnosis (for example, retinal data of a subject) and obtain diagnostic information predicted by the diagnostic data using a learned diagnostic model.

The storage module (21) may store a diagnostic model. The storage module (21) may store parameters, variables, etc. of the diagnostic model.

The storage module (21) may be implemented as a non-volatile semiconductor memory, hard disk, flash memory, RAM, Read Only Memory (ROM), Electrically Erasable Programmable Read-Only Memory (EEPROM), or other tangible non-volatile recording media.

The storage module (21) may store various processing programs, parameters for performing processing of the programs, or data of such processing results. As an example, the storage module (21) may store a data processing process program for performing diagnosis to be described later, a diagnostic process program, parameters for performing each program, and data obtained by performing such programs (for example, processed data or diagnostic result values). In addition, the storage module (21) may store various diagnostic models to be described later.

Although not shown, the diagnostic device (20) may further include an input module. The input module may obtain user input. For example, the input module may obtain user input requesting diagnostic information. In addition, the input module may obtain physical information of the subject (at least one of height, weight, age, sex, race, smoking status, blood pressure (for example, blood pressure level, presence or absence of hypertension), presence or absence of diabetes (or blood sugar level), cholesterol level). The processor (22) may obtain information input through the input module.

The diagnostic device (20) may further include a communication module (23). The communication module (23) may communicate with the learning device and/or the client device. As an example, the diagnostic device (20) may be provided in the form of a server that communicates with a client device. In this regard, it will be described in more detail below.

### 1.1.2.3. Server Device

According to one embodiment of the present invention, the diagnostic system may include a server device. The diagnostic system according to one embodiment of the present invention may include a plurality of server devices.

The server device may store and/or drive a diagnostic model. The server device may store weight values constituting a learned diagnostic model. The server device may collect or store data used for diagnosis.

The server device may output the result of a diagnostic process using a diagnostic model to a client device. The server device may obtain feedback from a client device. The server device may operate similarly to the diagnostic device described above.

FIG. 4 illustrates a diagnostic system according to one embodiment of the present invention. Referring to FIG. 4, the diagnostic system (20) according to one embodiment of the present invention may include a diagnostic server (40), a learning device, and a client device.

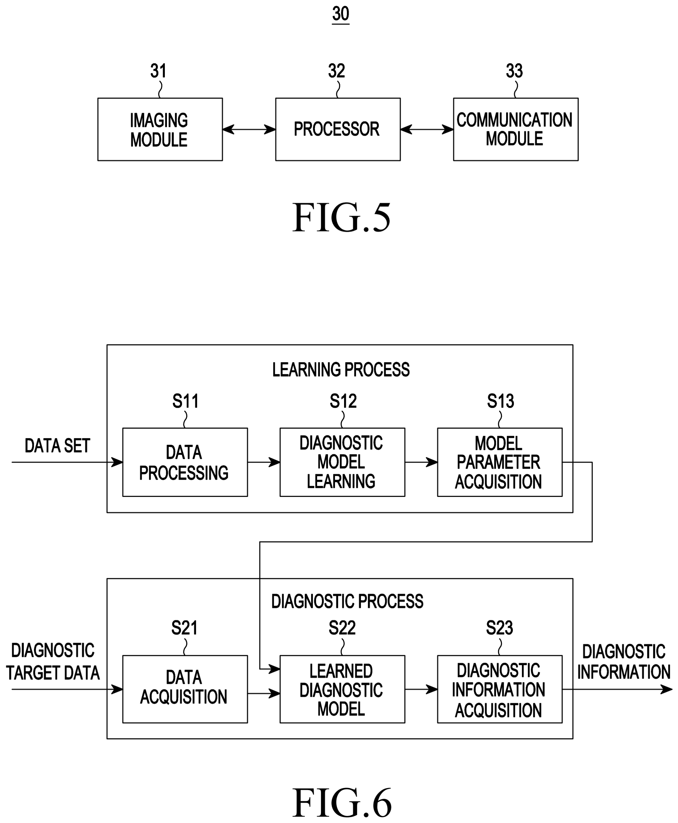

The diagnostic server (40), that is, the server device, may communicate with a plurality of learning devices or a plurality of diagnostic devices. Referring to FIG. 6, the diagnostic server (40) may communicate with a first learning device (10a) and a second learning device (10b). Referring to FIG. 4, the diagnostic server (40) may communicate with a first client device (30a) and a second client device (30b).

For example, the diagnostic server (40) may communicate with a first learning device (10a) that trains a first diagnostic model for obtaining first diagnostic information and a second learning device (10b) that trains a second diagnostic model for obtaining second diagnostic information.

The diagnostic server (40) may store a first diagnostic model for obtaining first diagnostic information and a second diagnostic model for obtaining second diagnostic information, obtain diagnostic information in response to a diagnostic information acquisition request from the first client device (30a) or the second client device (30b), and transmit the obtained diagnostic information to the first client device (30a) or the second client device (30b).

Alternatively, the diagnostic server (40) may communicate with a first client device (30a) that requests first diagnostic information and a second client device (30b) that requests second diagnostic information.

1.1.2.4. Client Device

The client device may request diagnostic information from a diagnostic device or a server device. The client device may obtain data necessary for diagnosis and transmit the obtained data to the diagnostic device.

FIG. 5 is a block diagram for explaining a client device according to one embodiment of the present invention. Referring to FIG. 5, the client device (30) according to one embodiment of the present invention may include an imaging module (31), a processor (32), and a communication module (33).

The imaging module (31) may obtain image or video data. The imaging module (31) may obtain a retinal image. However, the client device (30) may be replaced with another form of data acquisition unit other than the imaging module (31).

The communication module (33) may communicate with an external device, such as a diagnostic device or a server device. The communication module (33) may perform wired or wireless communication.

The processor (32) may control the imaging module (31) to obtain an image or data. The processor (32) may control the imaging module (31) to obtain a retinal image. The processor (32) may transmit the obtained retinal image to a diagnostic device. The processor (32) may transmit an image obtained through the imaging module (31) to a server device through the communication module (33) and obtain diagnostic information generated based thereon.

Although not shown, the client device may further include an output module. The output module may include a display that outputs video or images or a speaker that outputs audio. The output module may output video or image data obtained by the obtained imaging unit. The output module may output diagnostic information obtained from a diagnostic device.

Although not shown, the client device may further include an input module. The input module may obtain user input. For example, the input module may obtain user input requesting diagnostic information. The input module may obtain user information for evaluating diagnostic information obtained from a diagnostic device. In addition, the input module may obtain physical information of the subject (at least one of height, weight, age, sex, race, smoking status, blood pressure (for example, blood pressure level, presence or absence of hypertension), presence or absence of diabetes (or blood sugar level), cholesterol level).

In addition, although not shown, the client device may further include a storage module. The storage module may store images obtained by the imaging unit.

1.1.3. Overview of Diagnostic Process

A diagnostic process may be performed by the diagnostic system or diagnostic device disclosed in the present specification. The diagnostic process can be largely divided into a learning process for learning a diagnostic model used for diagnosis and a diagnostic process using the diagnostic model.

FIG. 6 is a diagram for explaining a diagnostic process according to one embodiment of the present invention.

Referring to FIG. 6, the diagnostic process according to one embodiment of the present invention may include a learning process of obtaining and processing data (S11), learning a diagnostic model (S12), and obtaining parameters of the learned diagnostic model (S13), and a diagnostic process of obtaining diagnostic target data (S21) and obtaining diagnostic information (S23) using the diagnostic model (S22) learned based on the diagnostic target data.

More specifically, the learning process may include a data processing process of processing input learning image data to process it into a state that can be used for training the model, and a learning process of training the model using the processed data. The learning process may be performed by the learning device described above.

The diagnostic process may include a data processing process of processing input examination target image data to process it into a state capable of performing diagnosis using a diagnostic model, and a diagnostic process of performing diagnosis using the processed data. The diagnostic process may be performed by the diagnostic device or server device described above.

Hereinafter, each process will be described.

1.2. Learning Process

According to one embodiment of the present invention, a process for training a diagnostic model may be provided. As a specific example, a process for training a diagnostic model that performs or assists diagnosis based on retinal images may be disclosed.

The learning process described below may be performed by the learning device described above.

1.2.1. Learning Unit

According to one embodiment of the present invention, the learning process may be performed by a learning unit. The learning unit may be provided in the learning device described above.

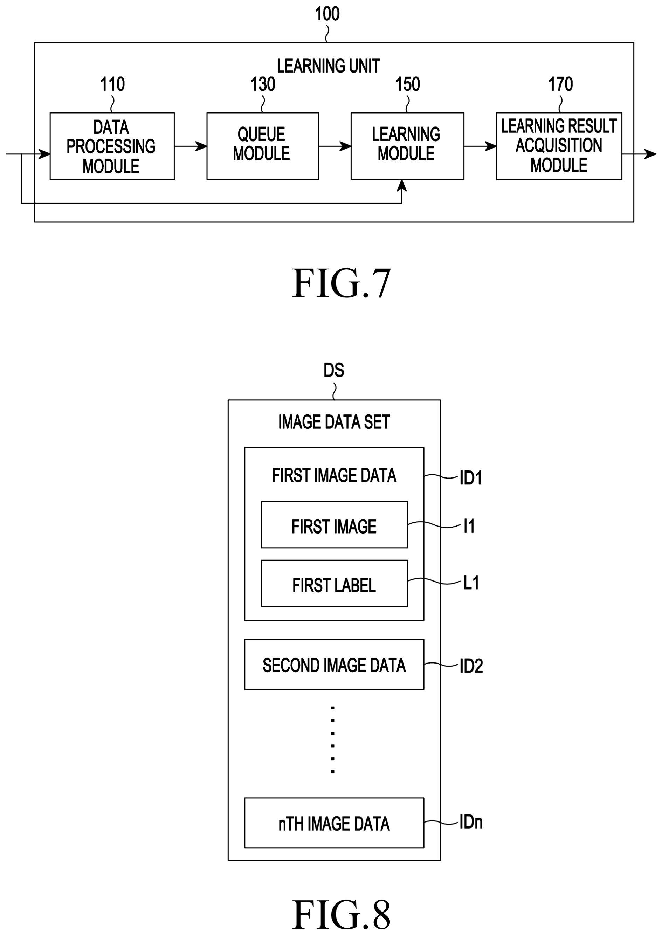

FIG. 7 is a diagram for explaining the configuration of a learning unit according to one embodiment of the present invention. Referring to FIG. 7, the learning unit (100) may include a data processing module (110), a queue module (130), a learning module (150), and a learning result acquisition module (170). Each module may perform individual steps of the data processing process and the learning process as described later. However, the components described in FIG. 7 and the functions performed by each component are not all essential, and depending on the learning form, some components may be added or some components may be omitted.

1.2.2. Data Processing Process

1.2.2.1. Image Data Acquisition

According to one embodiment of the present invention, a data set may be obtained. According to one embodiment of the present invention, a data processing module may obtain a data set.

The data set may be an image data set.

As an example, it may be a retinal image data set. The retinal image data set may be obtained using a general non-mydriatic retinal camera, etc. The retinal image may be a panoramic retinal image or a wide retinal image. The retinal image may be a red-free image. The retinal image may be an infrared-photographed image. The retinal image may be an autofluorescence-photographed image. The image data may be obtained in any one of JPG, PNG, DCM (DICOM), BMP, GIF, and TIFF formats.

As another example, the data set may be an image data set including any one of an Optical Coherence Tomography (OCT) image, an OCT angiography image, or a retinal angiography image. In this case, a diagnostic model learned using a data set including an OCT image, an OCT angiography image, or a retinal angiography image may predict or output diagnostic information (or label) based on a target OCT image, a target OCT angiography image, or a target retinal angiography image.

The data set may include a training data set. The data set may include a test data set. The data set may include a validation data set. In other words, the data set may be allocated to at least one data set among a training data set, a test data set, and a validation data set.

The data set may be determined in consideration of diagnostic information to be obtained using a diagnostic model learned through the corresponding data set. For example, when it is desired to train a diagnostic model for obtaining diagnostic information related to cataracts, the obtained data set may be determined as an infrared retinal image data set. Alternatively, when it is desired to train a diagnostic model for obtaining diagnostic information related to macular degeneration, the obtained data set may be an autofluorescence-photographed retinal image data set.

Individual data included in the data set may include a label. There may be a plurality of labels. In other words, individual data included in the data set may be labeled for at least one feature. As an example, the data set is a retinal image data set including a plurality of retinal image data, and each retinal image data may include a diagnostic information label (for example, presence or absence of a specific disease) and/or finding information (for example, presence or absence of abnormality in a specific site) label according to the corresponding image.

As another example, the data set is a retinal image data set and each retinal image data may include a peripheral information label for the corresponding image. For example, each retinal image data may include a peripheral information label including left-right eye information regarding whether the corresponding retinal image is an image of the left eye or the right eye, sex information regarding whether it is a retinal image of a female or a male, age information regarding the age of the subject who photographed the corresponding retinal image, etc.

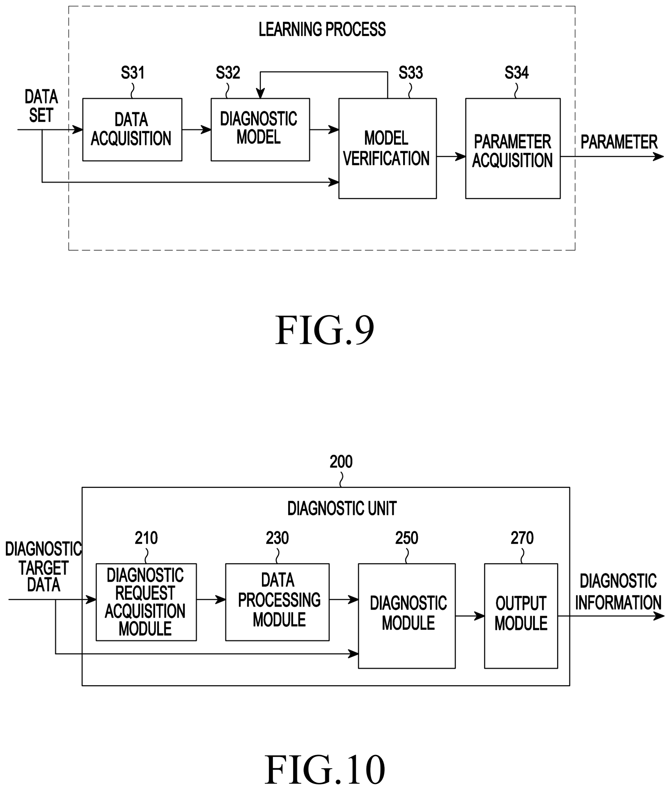

FIG. 8 is a conceptual diagram for explaining an image data set according to one embodiment of the present invention. Referring to FIG. 8, the image data set (DS) according to one embodiment of the present invention may include a plurality of image data (ID). Each image data (ID) may include an image (I) and a label (L) assigned to the image. Referring to FIG. 10, the image data set (DS) may include first image data (ID1) and second image data (ID2). The first image data (ID1) may include a first image (11) and a first label (L1) corresponding to the first image.

In FIG. 8, the case where one image data includes one label is described as a reference, but as described above, one image data may include a plurality of labels.

1.2.2.2. Image Preprocessing

According to one embodiment of the present invention, preprocessing of images may be performed. If images are used for learning as inputted, overfitting phenomena for unnecessary characteristics may occur and learning efficiency may also be degraded.

To prevent this, the data processing module may improve learning efficiency and performance by appropriately preprocessing image data to conform to the purpose of learning.

In one embodiment, the data processing module may perform preprocessing of images using or appropriately combining various techniques such as image resizing, grayscale conversion, histogram equalization, normalization, feature enhancement, image augmentation, noise removal, edge detection, segmentation, morphological operations, color space conversion, etc. Hereinafter, preprocessing of images using some techniques will be described.

1.2.2.2.1. Image Resizing

According to one embodiment of the present invention, the size of obtained image data may be adjusted. That is, images may be resized. According to one embodiment of the present invention, image resizing may be performed by the data processing module of the learning unit described above.

The size or aspect ratio of an image may be adjusted. A plurality of obtained images may have their sizes adjusted to have a constant size. Alternatively, images may have their sizes adjusted to have a constant aspect ratio. Resizing an image may be applying an image conversion filter to the image.

When the size or capacity of individual obtained images is excessively large or small, the size or capacity of the image may be adjusted to convert it to an appropriate size. Alternatively, when the sizes or capacities of individual images are various, the size or capacity may be unified through resizing.

According to one embodiment, the capacity of an image may be adjusted. For example, when the capacity of an image exceeds an appropriate range, the image may be reduced through down sampling. Alternatively, when the capacity of an image does not reach an appropriate range, the image may be enlarged through upsampling or interpolating.

According to another embodiment, the size or aspect ratio of an image may be adjusted by cropping the image or adding pixels to the obtained image. For example, when the image includes parts unnecessary for learning, a part of the image may be cropped to remove them. Alternatively, when a part of the image is cut off and the aspect ratio does not match, the image aspect ratio may be adjusted by adding columns or rows. In other words, the aspect ratio may be adjusted by adding margins or padding to the image.

According to yet another embodiment, the capacity and size or aspect ratio of an image may be adjusted together. As an example, when the capacity of an image is large, the image capacity may be reduced by down sampling the image, and unnecessary parts included in the reduced image may be cropped to convert it into appropriate image data.

In addition, according to another embodiment of the present invention, the orientation of image data may also be changed.

As a specific example, when a retinal image data set is used as a data set, each retinal image may have its capacity adjusted or size adjusted. Cropping may be performed to remove margin parts excluding the retina part of the retinal image, or padding may be performed to adjust the aspect ratio by supplementing cut parts of the retinal image.

1.2.2.2.2. Feature Enhancement

According to one embodiment of the present invention, the data processing module may perform preprocessing to emphasize features of retinal images. For example, the data processing module may perform preprocessing to facilitate detection of abnormal signs of ophthalmic disease in retinal images or preprocessing to emphasize retinal blood vessels or blood flow changes.

As an example, preprocessing of an image may be performed on an image for which the resizing processing described above has been completed. However, the content of the invention disclosed in the present specification is not limited thereto, and preprocessing on an image may be performed by omitting the resizing processing. Applying preprocessing to an image may be applying a preprocessing filter to the image.

According to one embodiment, a blur filter may be applied to an image. A Gaussian filter may be applied to an image. A Gaussian blur filter may be applied to an image. Alternatively, a deblur filter that sharpens an image may be applied to the image.

According to another embodiment, a filter that adjusts or modulates the color of an image may be applied. For example, a filter that changes the value of some components among RGB values constituting the image or that binarizes the image may be applied.

According to yet another embodiment, a filter that emphasizes a specific element in an image may be applied. For example, for retinal image data, preprocessing may be performed to emphasize vascular elements from each image. In this case, preprocessing to emphasize vascular elements may be applying one or more filters sequentially or in combination.

In addition, according to one embodiment of the present invention, preprocessing of an image may be performed in consideration of characteristics of diagnostic information to be obtained. For example, when it is desired to obtain diagnostic information related to findings such as retinal hemorrhage, drusen, microaneurysm, exudate, etc., preprocessing for converting the obtained retinal image into a red-free retinal image form may be performed.

1.2.2.2.3. Image Augmentation

According to one embodiment of the present invention, images may be augmented or expanded. Augmentation of images may be performed by the data processing module of the learning unit described above.

Augmented images may be used to improve training performance of a diagnostic model. For example, when the amount of data for training a diagnostic model is insufficient, the number of training image data may be increased by modulating existing training image data to expand the number of data for training and using the modulated (or changed) images together with original images. Accordingly, overfitting may be suppressed, the layers of the model may be formed deeper, and prediction accuracy may be improved.

For example, expansion of image data may be performed by horizontally flipping an image, cropping a part of an image, correcting color values of an image, or adding artificial noise. As a specific example, cropping a part of an image may be performed by cropping a partial region of elements constituting the image or randomly cropping partial regions. As more examples, image data may be expanded by horizontally flipping, vertically flipping, resizing by a certain ratio, cropping, padding, color adjusting, or brightness adjusting the image data.

In addition, in one embodiment, the data processing module may augment images by rotating retinal images. As the shape of the retina is circular, there may be no data loss even if the retina is rotated. Accordingly, when performing augmentation of images by rotating retinal images, a plurality of retinal images may be obtained without data loss. As an example, the data processing module may obtain a plurality of retinal images by rotating retinal images based on a predetermined angle (for example, 15 degrees, 30 degrees, 60 degrees, etc.).

In addition, in one embodiment, the augmentation or expansion of image data described above may generally be applied to a training data set. However, it may also be applied to other data sets, for example, a test data set, that is, a data set for testing a model after learning using training data and verification using verification data have been completed.

As a specific example, when a retinal image data set is used as a data set, an augmented retinal image data set may be obtained by randomly applying one or more processes among flipping, cropping, adding noise, or changing color of images to increase the number of data.

1.2.2.3. Image Serialization

According to one embodiment of the present invention, image data may be serialized (linearization). Images may be serialized by the data processing module of the learning unit described above. The serialization module may serialize preprocessed image data and transfer it to a queue module.

When image data is used as it is for learning, decoding is required because the image data has an image file form such as JPG, PNB, DCM, etc., and if learning is performed through decoding each time, the performance of model learning may be degraded. Accordingly, learning may be performed by serializing the image file without using it as it is for learning. Therefore, serialization of image data may be performed to improve learning performance and speed. The image data to be serialized may be image data to which one or more steps of the image resizing and image preprocessing described above have been applied, or may be image data to which neither has been processed.

Each image data included in the image data set may be converted into string form. Image data may be converted into binarized data form. In particular, image data may be converted into a data form suitable for being used in learning a diagnostic model. As an example, image data may be converted into TFRecord form for use in learning a diagnostic model using tensorflow.

As a specific example, when a retinal image set is used as a data set, the obtained retinal image set may be converted into TFRecord form and used for learning a diagnostic model.

### 1.2.2.4. Queue

A queue may be used to resolve data bottlenecks. The queue module of the learning unit described above may store image data in a queue and transfer it to a learning model module.

Particularly, when a learning process is performed using a Central Processing Unit (CPU) and a Graphic Processing Unit (GPU) together, by using a queue, bottlenecks between the CPU and GPU may be minimized, access to the database may be smoothed, and memory usage efficiency may be improved.

The queue may store data used for learning a diagnostic model. The queue may store image data. The image data stored in the queue may be image data to which at least one of the data processing processes (that is, resizing, preprocessing, and augmentation) described above has been processed, or may be an image in an obtained state as it is.

The queue may store image data, preferably serialized image data as described above. The queue may store image data and supply image data to a diagnostic model. The queue may transfer image data to a diagnostic model in batch size units.

The queue may provide image data. The queue may provide data to a learning module to be described later. As data is extracted from the learning module, the number of data accumulated in the queue may decrease.

As learning of the diagnostic model progresses, when the number of data stored in the queue decreases below a reference, the queue may request replenishment of data. The queue may request replenishment of a specific type of data. When replenishment of data is requested, the queue may replenish data in the queue.

The queue may be provided in system memory of the learning device. For example, the queue may be formed in Random Access Memory (RAM) of a central processing unit (CPU). In this case, the size, that is, capacity, of the queue may be determined according to the RAM capacity of the CPU. As the queue, a First In First Out (FIFO) queue, a primary queue, or a random queue may be used.

### 1.2.3. Learning Process

According to one embodiment of the present invention, a learning process of a diagnostic model may be disclosed.

According to one embodiment of the present invention, learning of a diagnostic model may be performed by the learning device described above. The learning process may be performed by the processor of the learning device described above. The learning process may be performed by the learning module of the learning unit described above.

FIG. 9 is a block diagram for explaining a learning process of a diagnostic model according to one embodiment of the present invention. Referring to FIG. 9, the learning process of a diagnostic model according to one embodiment of the present invention may be performed by obtaining data (S31), learning a diagnostic model (S32), verifying the learned model (S33), and obtaining variables of the learned model (S34).

### 1.2.3.1. Data Input

A data set for learning a diagnostic model may be obtained.

The obtained data may be an image data set processed by the data processing process described above. As an example, the data set may include retinal image data that has been size-adjusted, to which preprocessing filters have been applied, and which has been serialized after data augmentation.

In the learning stage of the diagnostic model, a training data set may be obtained and used. In the verification stage of the diagnostic model, a verification data set may be obtained and used. In the test stage of the diagnostic model, a test data set may be obtained and used. Each data set may include retinal images and labels.

The data set may be obtained from a queue. The data set may be obtained from the queue in batch size units. For example, when 60 is designated as the batch size, the data set may be extracted from the queue in units of 60. The size of the batch size may be limited by the RAM capacity of the GPU.

The data set may be randomly obtained from the queue to the learning module. The data set may be obtained in the order accumulated in the queue.

The learning module may extract by specifying the configuration of the data set obtained from the queue. As an example, the learning module may extract so that retinal image data having a left eye label and retinal image data having a right eye label of a specific subject are used together for learning.

The learning module may obtain a data set of a specific label from the queue. As an example, the learning module may obtain a retinal image data set having an abnormal diagnostic information label from the queue. The learning module may obtain a data set by specifying the ratio of the number of data according to labels from the queue. As an example, the learning module may obtain a retinal image data set from the queue such that the number of retinal image data having an abnormal diagnostic information label and the number of retinal image data having a normal diagnostic information label is 1 to 1.

### 1.2.3.2. Model Design

The diagnostic model may be designed as various models such as a neural network model, machine learning model, etc. In one embodiment, when the diagnostic model includes a neural network model, the network model may include a plurality of layers.

The neural network model may be implemented in the form of a classifier that generates diagnostic information. The classifier may perform binary classification or multi-classification. For example, the neural network model may be a binary classification model that classifies input data into normal or abnormal classes for target diagnostic information such as a specific disease or abnormal sign. Alternatively, the neural network model may be a multi-classification model that classifies input data into a plurality of grade classes for a specific characteristic (for example, the degree of disease progression). Alternatively, the neural network model may be implemented as a regression type model that outputs a specific numerical value related to a specific disease.

The neural network model may include a Convolutional Neural Network (CNN). As a CNN structure, at least one of AlexNet, LENET, NIN, VGGNet, ResNet, WideResnet, GoogleNet, FractaNet, DenseNet, FitNet, RitResNet, HighwayNet, MobileNet, and DeeplySupervisedNet may be used. The neural network model may be implemented using a plurality of CNN structures.

As an example, the neural network model may be implemented to include a plurality of VGGNet blocks. As a more specific example, the neural network model may be provided by combining a first structure in which a CNN layer having 64 filters of 3x3 size, a Batch Normalization (BN) layer, and a ReLu layer are sequentially combined, and a second block in which a CNN layer having 128 filters of 3x3 size, a ReLu layer, and a BN layer are sequentially combined.

The neural network model may include a max pooling layer following each CNN block, and may include a Global Average pooling (GAP) layer, a Fully Connected (FC) layer, and an activation layer (for example, sigmoid, softmax, etc.) at the end.

In addition, in another embodiment, when the diagnostic model includes a machine learning model, the machine learning model may include a linear regression model, a Cox proportional hazards model, etc.

### 1.2.3.3. Model Learning

The diagnostic model may be learned using a training data set.

The diagnostic model may be learned using a labeled data set. However, the learning process of the diagnostic model described in the present specification is not limited thereto, and the diagnostic model may be learned in an unsupervised form using unlabeled data.

Learning of the diagnostic model may be performed by obtaining a result value using a diagnostic model to which arbitrary weight values are assigned based on training image data, comparing the obtained result value with a label value of the training data, and performing backpropagation according to the error to optimize the weight values. In addition, learning of the diagnostic model may be influenced by a verification result of the model to be described later, a test result, and/or feedback from a diagnostic stage.

The learning of the diagnostic model described above may be performed using TensorFlow. However, the present invention is not limited thereto, and frameworks such as Theano, Keras, Caffe, Torch, and Microsoft Cognitive Toolkit (CNTK) may be used for learning the diagnostic model.

### 1.2.3.4. Model Validation

The diagnostic model may be verified using a verification data set. Verification of the diagnostic model may be performed by obtaining a result value for the verification data set from the diagnostic model on which learning has been performed, and comparing the result value with a label of the verification data set. Verification may be performed by measuring accuracy of the result value. According to the verification result, parameters (for example, weights and/or biases) or hyper parameters (for example, learning rate) of the diagnostic model may be adjusted.

As an example, the learning device according to one embodiment of the present invention may train a diagnostic model that predicts diagnostic information based on retinal images, and perform verification of the diagnostic model by comparing diagnostic information for a verification retinal image of the learned model with a verification label corresponding to the verification retinal image.

For verification of the diagnostic model, a separate verification set (external data set), that is, a data set having distinguishing factors not included in the training data set, may be used. For example, the separate verification set may be a data set that is distinguished from the training data set by factors such as race, environment, age, sex, etc.

### 1.2.3.5. Model Testing

The diagnostic model may be tested using a test data set.

According to the learning process according to one embodiment of the present invention, the diagnostic model may be tested using a test data set that is distinguished from the training data set and the verification data set. According to the test result, parameters (for example, weights and/or biases) or hyper parameters (for example, learning rate) of the diagnostic model may be adjusted.

As an example, the learning device according to one embodiment of the present invention may obtain a result value using test retinal image data not used for training and verification as input from a diagnostic model learned to predict diagnostic information based on retinal images, and perform testing of the learned and verified diagnostic model.

For testing the diagnostic model, a separately provided verification set (external data set), that is, a data set having factors distinguished from training and/or verification data, may be used.

### 1.2.3.6. Output of Results

As a result of learning the diagnostic model, optimized parameter values of the model may be obtained. As described above, as learning of the model is repeatedly performed using the test data set, more appropriate parameter (or variable) values may be obtained. When learning has progressed sufficiently, optimized values of weights and/or biases may be obtained.

According to one embodiment of the present invention, the learned diagnostic model and/or parameters or variables of the learned diagnostic model may be stored in the learning device and/or the diagnostic device (or server). The learned diagnostic model may be used for prediction of diagnostic information by the diagnostic device and/or the client device. In addition, parameters or variables of the learned diagnostic model may be updated by feedback obtained from the diagnostic device or the client device.

### 1.2.3.7. Model Ensemble

According to one embodiment of the present invention, in the process of learning one diagnostic model, a plurality of sub-models may be learned simultaneously. The plurality of sub-models may have different hierarchical structures.

In this case, the diagnostic model according to one embodiment of the present invention may be implemented by combining a plurality of sub-diagnostic models. In other words, learning of the diagnostic model may be performed using an ensemble technique that combines a plurality of sub-diagnoses.

When a diagnostic model is constructed by forming an ensemble, prediction may be performed by synthesizing results predicted from various types of sub-diagnostic models, so that accuracy of result prediction may be further improved.

### 1.3. Diagnostic Process

According to one embodiment of the present invention, a diagnostic process (or diagnostic process) for obtaining diagnostic information using a diagnostic model may be provided. As a specific example, by the diagnostic process, diagnostic information (for example, diagnostic information or finding information) may be predicted using a retinal image and through a learned diagnostic model.

The diagnostic process described below may be performed by a diagnostic device.

### 1.3.1. Diagnostic Unit

According to one embodiment of the present invention, the diagnostic process may be performed by the processor of the diagnostic device described above. The processor may be provided in the diagnostic device described above.

FIG. 10 is a diagram for explaining the configuration of a diagnostic unit according to one embodiment of the present invention. Referring to FIG. 10, the diagnostic unit (200) may include a diagnostic request acquisition module (210), a data processing module (230), a diagnostic module (250), and an output module (270).

Each module may perform individual steps of the data processing process and the learning process as described later. However, the components described in FIG. 10 and the functions performed by each component are not all essential, and some components may be added or some components may be omitted depending on the aspect of diagnosis.

1.3.2. Data Acquisition and Diagnostic Request

The diagnostic device according to one embodiment of the present invention may obtain diagnostic target data and obtain diagnostic information based thereon. The diagnostic target data may be image data. Data acquisition and acquisition of a diagnostic request may be performed by the diagnostic request acquisition module of the diagnostic unit described above.

As an example, the diagnostic target data (TD) may include a diagnostic target image (TI) and diagnostic target object information (PI; patient information).

The diagnostic target image (TI) may be an image for obtaining diagnostic information regarding a diagnostic target object. For example, the diagnostic target image may be a retinal image and/or a retinal image. The diagnostic target (TI) may have any one format of JPG, PNG, DCM (DICOM), BMP, GIF, and TIFF.

The diagnostic object information (PI) may be information for identifying a diagnostic target object. Alternatively, the diagnostic object information (PI) may be characteristic information of the diagnostic target object or image. For example, the diagnostic object information (PI) may include information such as the imaging date and time of the diagnostic target image, imaging equipment, identification number of the diagnostic target subject, ID, name, sex, age, weight, race, smoking status, blood pressure (presence or absence of hypertension), presence or absence of diabetes, etc. When the diagnostic target image is a retinal image, the diagnostic object information (PI) may further include ocular-related information such as binocular information indicating whether it is the left eye or the right eye.

The diagnostic device may obtain a diagnostic request. The diagnostic device may obtain diagnostic target data together with the diagnostic request. When a diagnostic request is obtained, the diagnostic device may obtain diagnostic information using a learned diagnostic model. The diagnostic device may obtain a diagnostic request from a client device. Alternatively, the diagnostic device may obtain a diagnostic request from a user through a separately provided input means.

1.3.3. Data Processing Process

The obtained data may be processed. Data processing may be performed by the data processing module of the diagnostic unit described above.

The data processing process may generally be performed similarly to the data processing process in the learning process described above. Hereinafter, the data processing process in the diagnostic process will be described focusing on differences from the data processing process in the learning process.

In the diagnostic process, the diagnostic device may obtain data as in the learning process. In this case, the obtained data may be in the same format as the data obtained in the learning process. For example, when the learning device trains a diagnostic model using image data in DCM format in the learning process, the diagnostic device may obtain a DCM image and obtain diagnostic information using the learned diagnostic model.

In the diagnostic process, the obtained diagnostic target image may be resized similarly to the image data used in the learning process. The diagnostic target image may have its form adjusted to have an appropriate capacity, size, and/or aspect ratio in order to efficiently perform prediction of diagnostic information through a learned diagnostic model.

For example, when the diagnostic target image is a retinal image, resizing such as cropping unnecessary parts of the image or reducing its size may be performed for prediction of diagnostic information based on the retinal image.

In the diagnostic process, a preprocessing filter may be applied to the obtained diagnostic target image, similarly to the image data used in the learning process. An appropriate filter may be applied to the diagnostic target image so that accuracy of diagnostic information prediction through a learned diagnostic model is further improved.

For example, when the diagnostic target image is a retinal image, preprocessing that facilitates prediction of diagnostic information, such as image preprocessing that emphasizes blood vessels or image preprocessing that emphasizes or weakens specific colors, may be applied to the diagnostic target image.

In the diagnostic process, the obtained diagnostic target image may be serialized similarly to the image data used in the learning process. The diagnostic target image may be converted or serialized into a form that facilitates driving the diagnostic model in a specific work frame.

Serialization of the diagnostic target image may be omitted. This may be because, unlike in the learning stage, in the diagnostic stage, the number of data processed by the processor at one time is not large, so the burden on data processing speed is relatively small.

In the diagnostic process, the obtained diagnostic target image may be stored in a queue similarly to the image data used in the learning process. However, since the number of processing data in the diagnostic process is smaller than in the learning process, the step of storing data in a queue may be omitted.

Meanwhile, in the diagnostic process, since an increase in the number of data is not required, data augmentation or image augmentation procedures may not be used, unlike in the learning process, for obtaining accurate diagnostic information.

1.3.4. Diagnostic Process

According to one embodiment of the present invention, a diagnostic process using a learned diagnostic model may be disclosed. The diagnostic process may be performed in the diagnostic device described above. The diagnostic process may be performed in the diagnostic server described above. The diagnostic process may be performed in the control unit of the diagnostic device described above. The diagnostic process may be performed by the diagnostic module of the diagnostic unit described above.

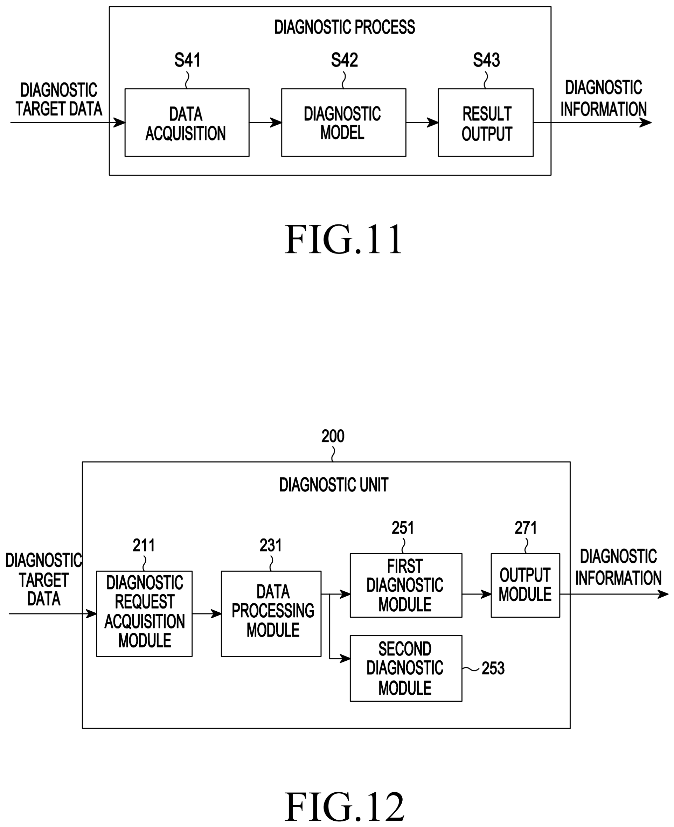

FIG. 11 is a diagram for explaining a diagnostic process according to one embodiment of the present invention. Referring to FIG. 11, the diagnostic process may be performed by obtaining diagnostic target data (S31), using a learned diagnostic model (S42), and obtaining a result corresponding to the obtained diagnostic target data (S43). However, data processing may be selectively performed.

Hereinafter, referring to FIG. 11, each step of the diagnostic process will be described.

1.3.4.1. Data Input

According to one embodiment of the present invention, the diagnostic module may obtain diagnostic target data. The obtained data may be processed data as described above. As an example, the obtained data may be retinal image data of a subject to which preprocessing has been applied to adjust size and emphasize blood vessels. According to one embodiment of the present invention, a left eye image and a right eye image of one subject may be input together as diagnostic target data.

1.3.4.2. Data Classification

A diagnostic model provided in classifier form may classify an input diagnostic target image into a positive or negative class for a predetermined label.

The learned diagnostic model may receive diagnostic target data as input and output a predicted label. The learned diagnostic model may output a predicted value of diagnostic information. Diagnostic information may be obtained using the learned diagnostic model. Diagnostic information may be determined based on a predicted label.

For example, the diagnostic model may predict diagnostic information (that is, information regarding the presence or absence of disease) or finding information (that is, information regarding the presence or absence of abnormal findings) regarding an ophthalmic disease or systemic disease of a subject. In this case, diagnostic information or finding information may be output in probability form. For example, the probability that a subject has a specific disease or the probability that a retinal image of a subject has specific abnormal findings may be output. When using a diagnostic model provided in classifier form, a predicted label may be determined by considering whether an output probability value (or prediction score) exceeds a threshold value.

As a specific example, the diagnostic model may output the presence or absence of diabetic retinopathy of a subject as a probability value using the retinal image of the subject as a diagnostic target image. When using a diagnostic model in classifier form with 1 as normal, a retinal photograph of a subject may be input to the diagnostic model, and regarding whether the subject has diabetic retinopathy, a probability value of normal : abnormal may be obtained in a form such as 0.74:0.26.

Here, the case of classifying data using a diagnostic model in classifier form has been described as a reference, but the present invention is not limited thereto, and a specific diagnostic value (for example, blood pressure, etc.) may be predicted using a diagnostic model implemented in regression model form.

According to another embodiment of the present invention, suitability information of an image may be obtained. The suitability information may indicate whether a diagnostic target image is suitable for obtaining diagnostic information using a diagnostic model.

The suitability information of an image may be quality information of an image. Quality information or suitability information may indicate whether a diagnostic target image reaches a reference level.

For example, when a diagnostic target image has defects due to defects in photographing equipment or the influence of lighting during photographing, an unsuitable result may be output as suitability information for the corresponding diagnostic target image. When a diagnostic target image includes noise above a certain level, the diagnostic target image may be determined to be unsuitable.

The suitability information may be a value predicted using a diagnostic model. Alternatively, the suitability information may be information obtained through a separate image analysis process.

According to one embodiment, even when an image is classified as unsuitable, diagnostic information obtained based on the unsuitable image may be obtained.

According to one embodiment, an image classified as unsuitable may be re-examined by a diagnostic model.

In this case, the diagnostic model performing re-examination may be different from the diagnostic model performing initial examination. For example, the diagnostic device may store a first diagnostic model and a second diagnostic model, and an image classified as unsuitable through the first diagnostic model may be examined through the second diagnostic model.

According to yet another embodiment of the present invention, a map may be obtained from a learned diagnostic model. Diagnostic information may include a map. A map may be obtained together with other diagnostic information. For example, a map may include a Saliency Map, a Class Activation Map (CAM), a Heat Map, etc. In addition, in the case of CAM, it may be selectively obtained. For example, in the case of CAM, CAM may be extracted and/or output when diagnostic information or finding information obtained by a diagnostic model is classified into an abnormal class.

1.3.5. Output of Diagnostic Information

Diagnostic information may be determined based on results output from a diagnostic model. Output of diagnostic information may be performed by the output module of the diagnostic unit described above. Diagnostic information may be output from a diagnostic device to a client device. Diagnostic information may be output from a diagnostic device to a server device. Diagnostic information may be stored in a diagnostic device or diagnostic server. Diagnostic information may be stored in a separately provided server device, etc.

Diagnostic information may be managed in database form. For example, obtained diagnostic information may be stored and managed together with a diagnostic target image of the corresponding subject according to an identification number of the subject. In this case, the diagnostic target image and diagnostic information of the subject may be managed in chronological order. By managing diagnostic information and diagnostic target images in time series, tracking and history management of diagnostic information for each individual may be facilitated.

Diagnostic information may be provided to a user. Diagnostic information may be provided to a user through output means of a diagnostic device or client device. Diagnostic information may be output so that a user can recognize it through visual or auditory output means provided in a diagnostic device or client device.

According to one embodiment of the present invention, an interface for effectively providing diagnostic information to a user may be provided.

In addition, when an image is classified as unsuitable, suitability information of the image may be provided together. As an example, when an image is classified as unsuitable, diagnostic information obtained according to the corresponding image and unsuitability determination information may be provided together.

A diagnostic target image determined to be unsuitable may be classified as a rephotographing target image. In this case, rephotographing guidance for the target object of the image classified as a rephotographing target may be provided together with suitability information. Meanwhile, in response to providing diagnostic information obtained through a diagnostic model, feedback related to learning the diagnostic model may be obtained. For example, feedback for adjusting parameters or hyper parameters related to learning the diagnostic model may be obtained. Feedback may be obtained through an input module provided in a diagnostic device or client device.

According to one embodiment of the present invention, diagnostic information corresponding to a diagnostic target image may include grade information. Grade information may be selected from a plurality of grades. Grade information may be determined based on diagnostic information and/or finding information obtained through a diagnostic model. Grade information may be determined in consideration of suitability information or quality information of a diagnostic target image. When the diagnostic model is a classifier model that performs multi-classification, grade information may be determined in consideration of the class into which the diagnostic target image is classified by the diagnostic model. When the diagnostic model is a regression model that outputs a numerical value related to a specific disease, grade information may be determined in consideration of the output numerical value. In addition, grade information may be determined by applying a predetermined cut-off value to a score according to diagnostic information.

For example, diagnostic information obtained corresponding to a diagnostic target image may include any one grade information selected from first grade information or second grade information. Grade information may be selected as first grade information when abnormal finding information or abnormal diagnostic information is obtained through a diagnostic model. Grade information may be selected as second grade information when abnormal finding information or abnormal diagnostic information is not obtained through a diagnostic model. Alternatively, grade information may be selected as first grade information when a numerical value obtained through a diagnostic model exceeds a reference numerical value, and may be selected as second grade information when the obtained numerical value does not reach the reference numerical value. First grade information may indicate that stronger abnormal information exists in the diagnostic target image compared to second grade information.

Meanwhile, grade information may be selected as third grade information when it is determined using image analysis or a diagnostic model that the quality of the diagnostic target image is below a reference. Alternatively, diagnostic information may include third grade information together with first or second grade information.

1.4. Diagnostic System Using Multiple Diagnostic Models

According to one embodiment of the present invention, diagnostic information may be output using a diagnostic model as described above. The diagnostic model described above may be composed of one diagnostic model or may be composed of a plurality of diagnostic models. And, when the diagnostic model is composed of a plurality of diagnostic models, the plurality of diagnostic models may be configured in parallel or may be configured in series. Hereinafter, parallel diagnostic models and serial diagnostic models will be described.

1.4.1.1. Parallel Diagnostic System Configuration

According to one embodiment of the present invention, a parallel diagnostic system for obtaining a plurality of diagnostic information may be provided. The parallel diagnostic system may train a plurality of diagnostic models for obtaining a plurality of diagnostic information, and obtain a plurality of diagnostic information using the plurality of learned diagnostic models.

For example, the parallel diagnostic system may train a first diagnostic model that obtains first diagnostic information related to the presence or absence of ophthalmic disease of a subject and a second diagnostic model that obtains second diagnostic information related to the presence or absence of systemic disease of a subject based on retinal images, and output diagnostic information regarding the presence or absence of ophthalmic disease and the presence or absence of systemic disease of a subject using the learned first diagnostic model and second diagnostic model.

The plurality of diagnostic models may be learned in parallel and/or independently. By learning models to predict different labels through a plurality of diagnostic models in this way, prediction accuracy for each label may be improved and efficiency of prediction operations may be increased. Since matters previously described may be applied to learning of a plurality of diagnostic models, detailed description will be omitted.

1.4.1.2. Parallel Diagnostic Process

According to one embodiment of the present invention, a diagnostic process for obtaining a plurality of diagnostic information may be provided. The diagnostic process for obtaining a plurality of diagnostic information may be implemented in the form of a parallel diagnostic process including a plurality of mutually independent diagnostic processes.

According to one embodiment of the present invention, the diagnostic process may be performed by a plurality of diagnostic modules. Each diagnostic process may be performed independently.

FIG. 12 is a block diagram for explaining a diagnostic unit according to one embodiment of the present invention.

Referring to FIG. 12, the diagnostic unit (200) according to one embodiment of the present invention may include a diagnostic request acquisition module (211), a data processing module (231), a first diagnostic module (251), a second diagnostic module (253), and an output module (271). Each module of the diagnostic unit (200) may operate similarly to the diagnostic module of the diagnostic unit shown in FIG. 10 unless otherwise specifically mentioned.

In FIG. 12, even when the diagnostic unit (200) includes a plurality of diagnostic modules, the diagnostic request acquisition module (211), data processing module (231), and output module (271) are shown as common, but the present invention is not limited to such a configuration, and the diagnostic request acquisition module, data processing module, and/or output module may also be provided in plurality. The plurality of diagnostic request acquisition modules, data processing modules, and/or output modules may also operate in parallel.

For example, the diagnostic unit (200) may include a first data processing module that performs first processing on an input diagnostic target image and a second processing module that performs second data processing on the diagnostic target image, and the first diagnostic module may obtain first diagnostic information based on the first processed diagnostic target image, and the second diagnostic module may obtain second diagnostic information based on the second processed diagnostic target image. The first processing and/or second processing may be any one selected from image resizing, color modulation of image, blur filter application, blood vessel emphasis processing, red-free conversion, partial region cropping, and partial element extraction.

The plurality of diagnostic modules may obtain different diagnostic information. The plurality of diagnostic modules may obtain diagnostic information using different diagnostic models. For example, the first diagnostic module may obtain first diagnostic information related to whether a subject corresponds to ophthalmic disease using a first diagnostic model that predicts whether the subject corresponds to ophthalmic disease, and the second diagnostic module may obtain second diagnostic information related to whether the subject corresponds to systemic disease using a second diagnostic model that predicts whether the subject corresponds to systemic disease.

As a more specific example, the first diagnostic module may obtain first diagnostic information regarding whether a subject corresponds to diabetic retinopathy using a first diagnostic model that predicts whether the subject corresponds to diabetic retinopathy based on retinal images, and the second diagnostic module may obtain second diagnostic information related to whether the subject corresponds to hypertension using a second diagnostic model that predicts whether the subject corresponds to hypertension based on retinal images.

In addition, the diagnostic process according to one embodiment of the present invention may include a plurality of sub-diagnostic processes. Each sub-diagnostic process may be performed using different diagnostic models. Each sub-diagnostic process may be performed in different diagnostic processes. For example, the first diagnostic module may perform a first sub-diagnostic process for obtaining first diagnostic information through a first diagnostic model. Alternatively, the second diagnostic module may perform a second sub-diagnostic process for obtaining second diagnostic information through a second diagnostic model.

The plurality of learned diagnostic models may receive diagnostic target data as input and output predicted labels or probabilities. Each diagnostic model may be provided in classifier form and may classify input diagnostic target data for a predetermined label. In this case, the plurality of diagnostic models may be provided in classifier form learned for different characteristics.

Meanwhile, a map may be obtained from each diagnostic model, and the map may be selectively obtained. The map may be extracted when predetermined conditions are satisfied. For example, when the first diagnostic information indicates that the subject is abnormal for the first characteristic, a first map may be obtained from the first diagnostic model.

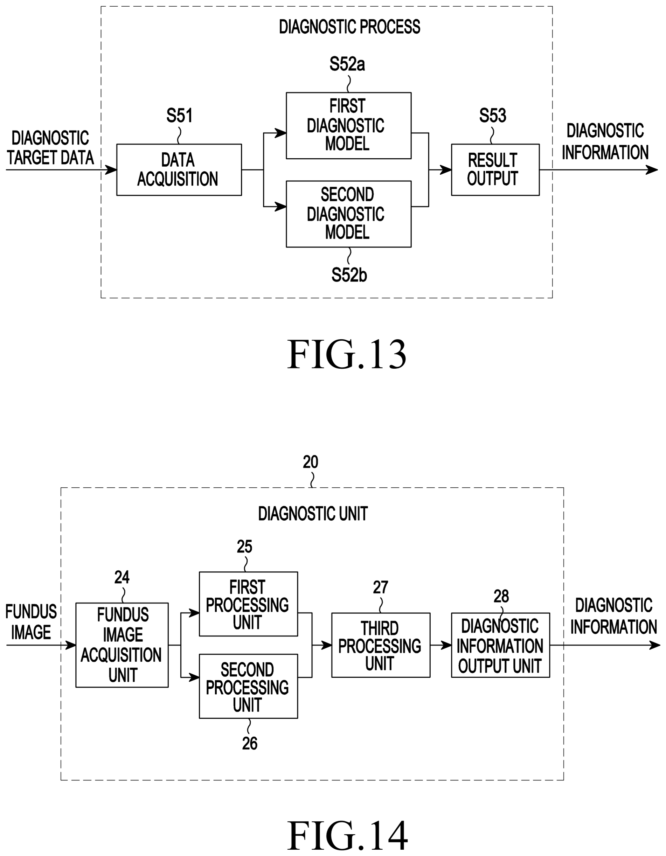

FIG. 13 is a diagram for explaining a diagnostic process according to one embodiment of the present invention.

Referring to FIG. 13, the diagnostic process according to one embodiment of the present invention may include obtaining diagnostic target data (S51) and obtaining diagnostic information according to the diagnostic target data using a first diagnostic model and a second diagnostic model (S51a, S51b) (S53). The diagnostic target data may be processed data.

The diagnostic process according to one embodiment of the present invention may include obtaining first diagnostic information through a learned first diagnostic model and obtaining second diagnostic information through a learned second diagnostic model. The first diagnostic model and the second diagnostic model may obtain first diagnostic information and second diagnostic information, respectively, based on the same diagnostic target data.

For example, the first diagnostic model and the second diagnostic model may obtain first diagnostic information regarding ophthalmic disease of a subject and second diagnostic information regarding whether the subject has renal disease, respectively, based on a diagnostic target retinal image.

In addition, unless otherwise specifically mentioned, the diagnostic process described in relation to FIG. 13 may be implemented similarly to the diagnostic process described above in relation to FIG. 11.

1.4.1.3. Output of Diagnostic Information

According to one embodiment of the present invention, diagnostic information obtained by a parallel diagnostic process may be obtained. The obtained diagnostic information may be stored in a diagnostic device, server device, and/or client device. The obtained diagnostic information may be transferred to an external device.

The plurality of diagnostic information may respectively indicate a plurality of labels predicted by a plurality of diagnostic models. The plurality of diagnostic information may correspond, respectively, to a plurality of labels predicted by a plurality of diagnostic models. Alternatively, diagnostic information may be information determined based on a plurality of labels predicted by a plurality of diagnostic models. Diagnostic information may correspond to a plurality of labels predicted by a plurality of diagnostic models.

In other words, the first diagnostic information may be diagnostic information corresponding to a first label predicted through a first diagnostic model. Alternatively, the first diagnostic information may be diagnostic information determined by considering together a first label predicted through a first diagnostic model and a second label predicted through a second diagnostic model.

Meanwhile, map images obtained from a plurality of diagnostic models may be output. A map image may be output when predetermined conditions are satisfied. For example, in any one case where the first diagnostic information indicates that the subject is abnormal for the first characteristic or the second diagnostic information indicates that the subject is abnormal for the second characteristic, a map image obtained from the diagnostic model from which diagnostic information indicated abnormality was output may be output.

A plurality of diagnostic information and/or map images may be provided to a user. The plurality of diagnostic information, etc. may be provided to a user through output means of a diagnostic device or client device.

According to one embodiment of the present invention, diagnostic information corresponding to a diagnostic target image may include grade information. Grade information may be selected from a plurality of grades. Grade information may be determined based on a plurality of diagnostic information and/or finding information obtained through a diagnostic model. Grade information may be determined in consideration of suitability information or quality information of a diagnostic target image. Grade information may be determined in consideration of classes into which a diagnostic target image is classified by a plurality of diagnostic models. Grade information may be determined in consideration of numerical values output from a plurality of diagnostic models.

For example, diagnostic information obtained corresponding to a diagnostic target image may include any one grade information selected from first grade information or second grade information. Grade information may be selected as first grade information when, among diagnostic information obtained through a plurality of diagnostic models, at least one abnormal finding information or abnormal diagnostic information is obtained. Grade information may be selected as second grade information when, among diagnostic information obtained through diagnostic models, abnormal finding information or abnormal diagnostic information is not obtained.

Grade information may be selected as first grade information when at least one of numerical values obtained through a diagnostic model exceeds a reference numerical value, and may be selected as second grade information when all obtained numerical values do not reach the reference numerical value. First grade information may indicate that stronger abnormal information exists in the diagnostic target image compared to second grade information.

Grade information may be selected as third grade information when it is determined using image analysis or a diagnostic model that the quality of the diagnostic target image is below a reference. Alternatively, diagnostic information may include third grade information together with first or second grade information.

In addition, the diagnostic system according to one embodiment of the invention may include a retinal image acquisition unit, a first processing unit, a second processing unit, a third processing unit, and a diagnostic information output unit.

According to one embodiment of the present invention, the diagnostic system may include a diagnostic device. The diagnostic device may include a retinal image acquisition unit, a first processing unit, a second processing unit, a third processing unit, and/or a diagnostic information output unit. However, the present invention is not limited thereto, and each unit included in the diagnostic system may be respectively located at appropriate positions on a learning device, diagnostic device, learning diagnostic server, and/or client device. Hereinafter, for convenience, description will be made based on the case where the diagnostic device of the diagnostic system includes a retinal image acquisition unit, a first processing unit, a second processing unit, a third processing unit, and a diagnostic information output unit.

FIG. 14 is a diagram for explaining a diagnostic system according to one embodiment of the present invention. Referring to FIG. 14, the diagnostic system includes a diagnostic device, and the diagnostic device may include a retinal image acquisition unit, a first processing unit, a second processing unit, a third processing unit, and a diagnostic information output unit.

According to one embodiment of the present invention, a diagnostic system for assisting diagnosis of a plurality of diseases based on retinal images may include: a retinal image acquisition unit that obtains a target retinal image that serves as a basis for obtaining diagnostic information regarding a subject; a first processing unit that obtains a first result for the subject using a first diagnostic model-the first diagnostic model is machine-learned based on a first retinal image set-for the target retinal image; a second processing unit that obtains a second result for the subject using a second diagnostic model-the second diagnostic model is machine-learned based on a second retinal image set that is at least partially different from the first retinal image set-for the target retinal image; a third processing unit that determines diagnostic information regarding the subject based on the first result and the second result; and a diagnostic information output unit that provides the determined diagnostic information to a user.

The third processing unit may determine whether diagnostic information according to the target retinal image is normal information or abnormal information by considering the first result and the second result together.

The third processing unit may determine diagnostic information regarding the subject by giving priority to abnormal results so that diagnostic accuracy is improved.

The third processing unit may determine diagnostic information as normal when the first result is normal and the second result is normal, and may determine diagnostic information as abnormal when the first result is not normal or the second result is not normal.

The first result and the second result may be for the same disease or may be for different diseases.

At least one map related to a first/second result may be obtained through the first processing unit and/or the first/second diagnostic model, and the diagnostic information output unit may output an image of at least one map.

The diagnostic information output unit may output an image of at least one map when the diagnostic information obtained by the third processing unit is abnormal diagnostic information.

The diagnostic system may further include a fourth processing unit that obtains quality information of the target retinal image, and the diagnostic information output unit may output quality information of the target retinal image obtained by the fourth processing unit.

When it is determined in the fourth processing unit that quality information of the target retinal image is below a predetermined quality level, the diagnostic information output unit may provide information indicating that quality information of the target retinal image is below the predetermined quality level together with the determined diagnostic information to the user.

1.4.2.1. Configuration of Serial Diagnostic System

According to one embodiment, a serial diagnostic system in a form in which a plurality of diagnostic models are connected in series may be provided. Hereinafter, some embodiments of serial type diagnostic models will be described.

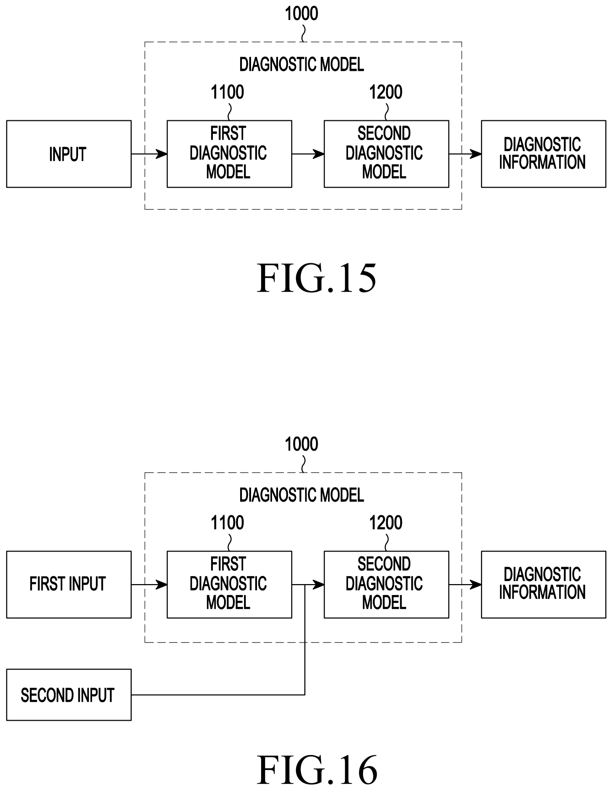

FIG. 15 is a diagram for explaining a serial diagnostic model according to one embodiment. Referring to FIG. 15, the diagnostic model (1000) may include a first serial model (1100) and a second serial model (1200).

The first diagnostic model (1100) may obtain input data including a retinal image and obtain a first output (or intermediate output).

The first diagnostic model (1100) may obtain input data including retinal images and/or other medical diagnostic images and/or non-visual diagnostic data. The input data may include retinal images, OCT images, iris images, ocular angiography images, lung CT images, lung CT images, heart CT images, lung X-ray images, heart X-ray images, kidney X-ray images, other tomography images, MRI images, or X-ray images. The input data may include data representing the age, height, sex, smoking status, family history, etc. of a subject.

The first output may be a value obtained by an output layer of the first diagnostic model (1100). For example, the first diagnostic model (1100) is a classifier model and the first output may include output values at a plurality of nodes of the output layer of the first diagnostic model (1100). Also, for example, the first diagnostic model (1100) is a regression model and the first output may include a numerical value obtained by the first diagnostic model (1100).

The first output may be a value provided by some layers of the first diagnostic model (1100). For example, the first output may be a value obtained based on a value of the output layer of the first diagnostic model (1100). Alternatively, the first output may be a value obtained based on a value of a hidden layer of the first diagnostic model (1100).

According to one embodiment, the first output may be a value obtained by an activation function in the output layer of the first diagnostic model (1100). When the output layer of the first diagnostic model (1100) includes a plurality of nodes (or neurons), the first output may include an output value according to each of the plurality of nodes or a value obtained through a predetermined function (for example, summation) based on each output value.

The activation function may be any one of a sigmoid function, hyperbolic tangent function, Rectified Linear Unit (ReLu) function, PReLu, Leaky ReLU function, Identity Function, Exponential Linear Unit (ELU) function, and Maxout function.

The first output may be a feature map or feature value related to a target disease. The first output may be a probability map, saliency map, heat map, etc. related to a target disease. The second diagnostic model (1200) may be provided to obtain diagnostic information based on a feature map or feature value related to a target disease.

The first output may be a probability phenotype related to a target disease. For example, when the target disease is coronary artery disease and diagnostic information is numerical information related to the target coronary artery disease, the first output may be the probability that the subject corresponds to target coronary artery disease obtained based on an ocular image. The second diagnostic model (1200) may be provided to obtain diagnostic information regarding the target disease based on probability expression related to the target disease.

The second diagnostic model (1200) may obtain a second output (or diagnostic information) based on the first output. The second diagnostic model (1200) may be a diagnostic model (1000) learned to obtain the second output using the first output as input. The second output may be diagnostic information in various forms described in the present specification.

FIG. 16 is a diagram for explaining a serial diagnostic model according to another embodiment. Referring to FIG. 16, the diagnostic model (1000) may include a first diagnostic model (1100) and a second diagnostic model (1200).

The first diagnostic model (1100) may obtain input data including an ocular image and obtain a first output (intermediate output). The second diagnostic model (1200) may obtain diagnostic information based on the first output and a second input.

The second input may be the same input data as the first input. For example, the first diagnostic model (1100) may obtain a first output based on an ocular image, and the second diagnostic model (1200) may obtain diagnostic information based on the first output and the ocular image.

The second input may be input data obtained based on the first input. For example, the second input may be input data obtained by performing image processing on an ocular image. For example, the second input may be a black-and-white processed ocular image, an ocular image with emphasized blood vessels, a blood vessel image extracted from an ocular image, or an ocular image with blood vessels removed.

The second input may be input data at least partially different from the first input.

The second input may be image data different from the first input. The second input may include retinal images,

EP 4 725 391 A1

OCT images, iris images, ocular angiography images, lung CT images, lung CT images, heart CT images, lung X-ray images, heart X-ray images, kidney X-ray images, other tomography images, MRI images, or X-ray images.

The second input may include non-visual information regarding a subject. For example, the first diagnostic model (1100) may obtain a first output regarding a target disease based on an ocular image, and the second diagnostic model (1200) may obtain diagnostic information based on the first output and a second input (for example, physical information of the subject (for example, age, sex, race, smoking status, blood pressure (for example, blood pressure level, presence or absence of hypertension), presence or absence of diabetes (or blood sugar level), cholesterol level, family history, etc.).

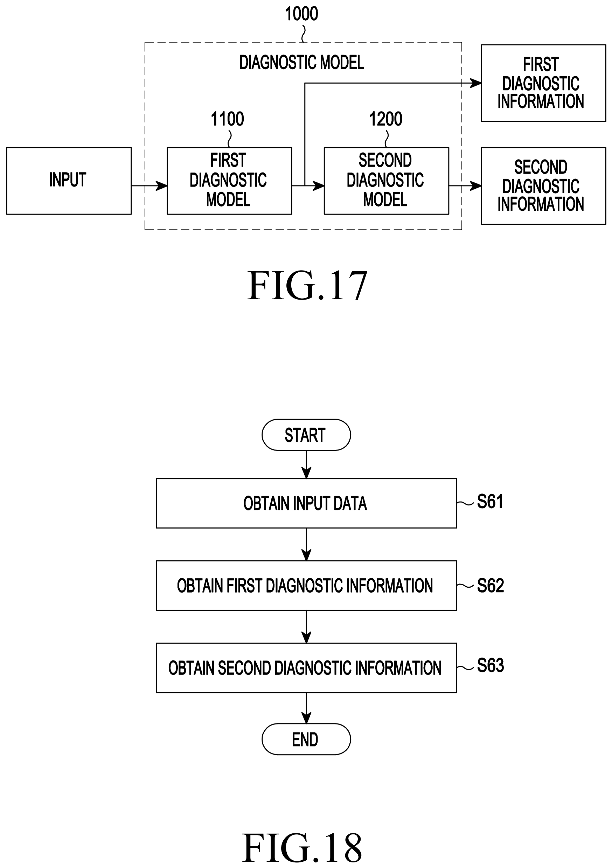

FIG. 17 is a diagram for explaining a serial diagnostic model according to yet another embodiment. Referring to FIG. 17, the diagnostic model (1000) may include a first diagnostic model (1100) and a second diagnostic model (1200).

Compared to FIG. 16, first diagnostic information obtained by the first diagnostic model (1100) may be further obtained by the diagnostic model (1000). The diagnostic model (1000) may obtain intermediate diagnostic information and secondary diagnostic information obtained based on the intermediate diagnostic information.

The diagnostic model (1000) may obtain first diagnostic information and second diagnostic information. The first diagnostic model (1100) may obtain input data including an ocular image and obtain a first output (first diagnostic information or intermediate output). The diagnostic model (1000) may obtain first diagnostic information based on the first output. The second diagnostic model (1200) may obtain second diagnostic information based at least in part on the first output. The diagnostic model (1000) may obtain second diagnostic information based at least in part on the first output and by considering together other information extracted from the ocular image.

The first diagnostic information and the second diagnostic information may be diagnostic information regarding the same target disease. The first diagnostic information may be diagnostic information that can be obtained (clinically or through a machine-learned model) based on ocular images, such as probability expressions for the presence or absence of ophthalmic disease, vascular abnormalities, presence or absence of renal disease, etc.

The second diagnostic information may represent more detailed diagnostic information than the first diagnostic information. For example, the second diagnostic information may include grade information representing the risk level for the target disease or score information representing a score related to the target disease, for the same target disease as the first diagnostic information.

The second diagnostic information may be associated with the first diagnostic information and may include diagnostic information that can be obtained by further considering information other than images, such as disease progression rate, guide information related to diagnostic information, etc.

Meanwhile, the first diagnostic information and the second diagnostic information may be diagnostic information regarding different diseases. The first diagnostic information and the second diagnostic information may be diagnostic information regarding different diseases belonging to the same group. For example, the first diagnostic information may be diagnostic information related to glaucoma belonging to the ophthalmic disease group and the second diagnostic information may be diagnostic information related to macular degeneration belonging to the ophthalmic disease group. For example, the first diagnostic information may be diagnostic information related to drusen belonging to the ophthalmic disease group and the second diagnostic information may be diagnostic information related to diabetic retinopathy belonging to the ophthalmic disease group.

The first diagnostic information and the second diagnostic information may be diagnostic information regarding different diseases belonging to different groups. For example, the first diagnostic information may be diagnostic information related to macular degeneration or drusen, etc. belonging to the ophthalmic disease group, and the second diagnostic information may be diagnostic information related to hyperlipidemia belonging to the cardiovascular disease group.

In the above embodiments, description has been made based on the case where the diagnostic model (1000) has a first diagnostic model (1100) and a second diagnostic model (1200), but the diagnostic model (1000) may include a greater number of diagnostic models. In addition, each diagnostic model may be connected through the parallel connection or serial connection described above.

1.4.2.2. Diagnosis Through Serial Diagnostic Model

According to one embodiment of the invention described in the present specification, a diagnostic method using a diagnostic model including serially connected sub-models may be provided.

FIG. 18 is a diagram for explaining a diagnostic method using a diagnostic model according to one embodiment.

Referring to FIG. 18, the diagnostic method according to one embodiment may include obtaining input data (S61), obtaining first diagnostic information (S62), and obtaining second diagnostic information (S63).

The step of obtaining input data (S61) may include obtaining a retinal image of a subject. The step of obtaining input data (S61) may further include obtaining a medical image for a body part other than the eye of the subject. The step of obtaining input data (S61) may further include obtaining non-visual information (for example, physical information of the subject) regarding the subject. The step of obtaining input data (S61) may further include performing preprocessing necessary for obtaining diagnostic information on an ocular image of the subject.

The step of obtaining first diagnostic information (S62) may include obtaining first diagnostic information regarding a subject based on an ocular image and through a first diagnostic model. Obtaining first diagnostic information may be obtaining diagnostic information related to a first disease. For example, obtaining first diagnostic information may include obtaining first diagnostic information representing the probability that a coronary artery calcium score of a subject is 0 or more based on an ocular image.

The step of obtaining second diagnostic information (S63) may include obtaining second diagnostic information regarding a subject based on the first diagnostic information and through a second diagnostic model.

Obtaining second diagnostic information may include obtaining diagnostic information that is related to a first disease and different from the first diagnostic information. For example, obtaining first diagnostic information may include obtaining first diagnostic information representing the probability that a coronary artery calcium score of a subject is 0 or more, and obtaining second diagnostic information may include obtaining second diagnostic information representing a score (for example, the probability of occurrence of cardiovascular disease-related events within 10 years) related to whether the subject has a target cardiovascular disease based on the first diagnostic information of the subject.

Alternatively, obtaining second diagnostic information may include obtaining diagnostic information related to a second disease different from the first disease. For example, obtaining first diagnostic information may include obtaining first diagnostic information representing whether a subject has a target ophthalmic disease, and obtaining second diagnostic information may include obtaining second diagnostic information representing whether the subject has a cardio-cerebrovascular disease.

Regarding diagnosis through the diagnostic model described above, it will be described in detail with specific examples below.

2. Method for Diagnosing Renal Disease

2.1.1. Biomarkers for Renal Disease

In one embodiment, renal disease biomarkers for predicting, measuring, and confirming the presence or absence of renal disease, degree of progression, etc. may be used in a method for diagnosing renal disease. In the present specification, renal disease biomarkers may include risk assessment tools for renal disease.

For example, renal disease biomarkers may include Estimated Glomerular Filtration Rate (eGFR) level, albuminuria level, cystatin C level, Kidney Disease: Improving Global Outcomes (KDIGO) grade, etc.

The eGFR level is calculated based on blood creatinine concentration, age, sex, and race, and can be used as an indicator for determining the filtration function of the kidney. As an example, based on the eGFR level, the risk of renal disease can be classified as low risk (90 mL/min/1.73m$^2$ or more), moderate risk (60-89 mL/min/1.73m$^2$), moderate-high risk (45-59 mL/min/1.73m$^2$), very-high risk (30-44 mL/min/1.73m$^2$), and highest risk (29 mL/min/1.73m$^2$ or less).

The albuminuria level relates to the ratio of albumin in urine and can be used as a major indicator for determining renal disease. The albuminuria level may be an albumin-to-creatinine ratio. As an example, based on the albuminuria level, the risk of renal disease can be classified as low risk (less than 30 mg/g), moderate risk (30-300 mg/g), and high risk (300 mg/g or more).

The cystatin C level may be a biomarker of kidney function measured in blood. The higher the cystatin C level, the higher the risk to kidney function can be determined. As an example, based on the cystatin C level, the risk of renal disease can be classified into a low-risk group (1.0 mg/L) and a high-risk group (exceeding 1.0 mg/L). Also, in another example, based on the cystatin C level, the risk of renal disease can be classified into a low-risk group (0.62-1.15 mg/L) and a high-risk group (exceeding 1.15 mg/L). Also, in yet another example, based on the cystatin C level, the risk of renal disease can be classified into a low-risk group (0.9 mg/L or less), a low-moderate risk group (0.9-1.2 mg/L), a moderate risk group (1.2-1.9 mg/L), a moderate-high risk group (1.9-3.0 mg/L), and a high-risk group (exceeding 3.0 mg/L).

The KDIGO grade may be a biomarker of kidney function determined by integrating the eGFR level (or grade) and the albuminuria level (or grade). As an example, the KDIGO grade can classify into a low-risk group, moderate risk group, and high-risk group by combining the eGFR level (or grade) and the albuminuria level (or grade).

In addition, various renal disease biomarkers may be used in methods for diagnosing renal disease.

2.1.2. Method for Diagnosing Renal Disease

The method for diagnosing renal disease according to one embodiment of the present specification may be performed using at least one of the one diagnostic model, parallel diagnostic model, or serial diagnostic model described above. Hereinafter, for convenience of explanation, description will be focused on performing the method for diagnosing renal disease using a serial diagnostic model.

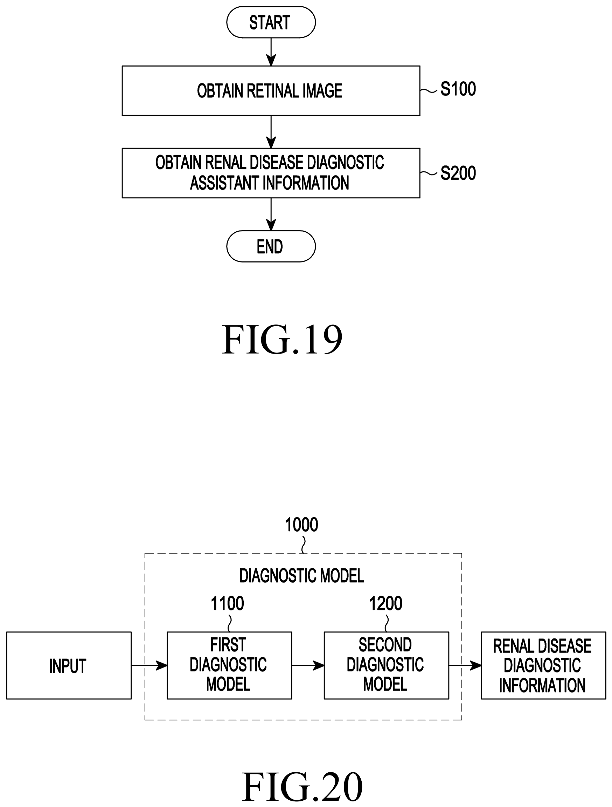

FIG. 19 is a diagram for explaining a method for diagnosing renal disease according to one embodiment.

Referring to FIG. 19, the processor of the diagnostic device may include obtaining a retinal image (S100) and obtaining renal disease diagnostic information (S200).

In step S100, the processor of the diagnostic device may obtain a retinal image. Also, according to the embodiment, the processor of the diagnostic device may perform preprocessing, augmentation, serialization, etc. on the obtained retinal image. Since the content described above can be applied to this, detailed description will be omitted.

Also, in step S200, the processor of the diagnostic device may obtain renal disease diagnostic information. In the present specification, renal disease diagnostic information may be expressed as Reti-CKD. The method for diagnosing renal disease will be described using FIG. 20.

FIG. 20 illustrates a diagnostic model for obtaining renal disease diagnostic information according to one embodiment.

Referring to FIG. 20, the diagnostic model (1000) may be included in the processor and/or storage module of the diagnostic device. Also, the description of the diagnostic models of FIGS. 15 to 18 may be applied to the diagnostic model (1000).

The diagnostic model (1000) may include a first diagnostic model (1100) and a second diagnostic model (1200). The first diagnostic model (1100) and the second diagnostic model (1200) may be machine learning models. Also, the first diagnostic model (1100) and the second diagnostic model (1200) may be models based on the same algorithm or may be models based on different algorithms. For example, the first diagnostic model (1100) and the second diagnostic model (1200) may be neural network models. Also, the first diagnostic model (1100) may be a neural network model, and the second diagnostic model (1200) may be a machine learning model that is not a neural network model. Hereinafter, for convenience of explanation, description will be focused on the case where the first diagnostic model (1100) is a neural network model and the second diagnostic model (1200) is a machine learning model that is not a neural network model, but the description of the present specification is not limited thereto.

The processor of the diagnostic device may input a retinal image (or preprocessed retinal image, serialized retinal image) to the first diagnostic model (1100). And the processor of the diagnostic device may obtain a probability value and/or grade that the subject of the retinal image is currently at high risk for renal disease from the first diagnostic model (1100) to which the retinal image has been input. For example, the processor of the diagnostic device may obtain a probability value and/or grade for the probability that the eGFR level of the subject of the current retinal image is less than or equal to 60 mL/min/1.73 m$^2$ and/or the probability that albuminuria is present in the urine of the current subject (or the probability that the albuminuria level is 300 mg/g or more) from the first diagnostic model (1100). Here, the probability value may be a number between 0 and 1.

Specifically, the first diagnostic model (1100) may be a neural network model of CNN structure.

In one embodiment, in the training process of the first diagnostic model (1100), the training target retinal images may include retinal images of patients with renal disease and retinal images of normal persons who are not patients with renal disease.

Also, in the training process of the first diagnostic model (1100), single images of each eye, the left eye and the right eye, of training target retinal images may be trained separately. In the training process, the ground truth may be the presence versus absence of renal disease. Also, predetermined information may be labeled for each retinal image. As an example, whether the eGFR level is 60 mL/min/1.73 m2 or less and/or whether albuminuria is present in the urine of the subject (for example, whether the albuminuria level is 300 mg/g or more) may be labeled as a binary variable for each retinal image. For example, if the eGFR level is 60 mL/min/1.73 m2 or less or albuminuria is present in the urine of the subject, the subject of the corresponding retinal image is labeled as having renal disease, and if the eGFR level is 60 mL/min/1.73 m2 or more and albuminuria is not present in the urine of the subject, the subject of the corresponding retinal image may be labeled as not having renal disease. Of course, the eGFR level and albuminuria level in the above example may be changed according to embodiments. Also, as another example, the eGFR level and albuminuria level of the subject may be labeled on each retinal image.

Also, in the evaluation process of the first diagnostic model (1100), probability values were assigned to each of the left eye retinal image and the right eye retinal image, and the average of the probability values may be regarded as the output of the test result.

Also, the last fully connected layer of the first diagnostic model (1100) may perform one logit probability prediction. The logit from the last fully connected layer may be converted to a probability using a sigmoid function. Also, the first diagnostic model (1100) may be trained to minimize the loss of targets and predictions. Also, in one embodiment, the first diagnostic model (1100) may be trained using an AdamW Optimizer for 50 epochs using a learning rate of 0.0002 and a schedule of cosine learning rate.

Also, for data augmentation of training target retinal images, at least one of mixup, cutmix, randaugment, enhancing contrast module, and random crop may be used. Also, focal loss and exponential moving average may be used for training the first diagnostic model (1100), and the size of training target retinal images may be set to 384x384.

Also, the processor of the diagnostic device may input the output value of the first diagnostic model (1100) and physical information of the subject (at least one of age, sex, race, smoking status, blood pressure (for example, blood pressure level, presence or absence of hypertension), presence or absence of diabetes (or blood sugar level), cholesterol level) to the second diagnostic model (1200). And the processor of the diagnostic device may obtain a probability value and/or grade for the probability of occurrence of renal disease-related events within 5 years of the subject from the second diagnostic model (1200). Here, renal disease-related events within 5 years may include the occurrence of fatal renal disease, the occurrence of non-fatal renal disease, and the occurrence of various events caused by renal disease (hospitalization, procedures, surgery, death, etc.) within 5 years from the time of taking the retinal image. Also, in some cases, the processor of the diagnostic device may obtain a probability value for the probability of occurrence of renal disease-related events within 5 years of the subject from the second diagnostic model (1200), and apply a predetermined cut-off value to the obtained probability value to obtain a grade corresponding to the obtained probability value. Also, although the present specification is described focusing on renal disease-related events within 5 years, it is not limited thereto, and according to embodiments, renal disease-related events of various periods such as within 3 years, within 10 years, etc. may be applied to the description of the present specification, of course.

Accordingly, the processor of the diagnostic device may output a probability value (score) and/or grade for the probability of occurrence of renal disease-related events within 5 years of the subject as renal disease diagnostic information.

Also, according to the embodiment, the processor of the diagnostic device may obtain a probability value and/or grade for the current risk of renal disease from the second diagnostic model (1200). Also, according to the embodiment, the processor of the diagnostic device may obtain a probability value and/or grade for the probability of occurrence of renal disease-related events within 5 years of the subject from the second diagnostic model (1200), and obtain a probability value and/or grade for the current risk of renal disease based on the obtained probability value and/or grade for the probability of occurrence of renal disease-related events within 5 years of the subject. For example, the processor of the diagnostic device may compare the probability value and/or grade for the probability of occurrence of renal disease-related events within 5 years of the subject obtained from the second diagnostic model (1200) with at least one predetermined reference probability value and/or grade, and obtain a probability value and/or grade for the current risk of renal disease based on the comparison result.

As an example, a first score and/or first grade of renal disease diagnostic information corresponding to high risk of existing biomarkers (for example, eGFR level of 44 mL/min/1.73m$^2$ or less, albuminuria level of 300 mg/g or more, and/or KDIGO grade high-risk group or more, etc.) may be predetermined. And the processor of the diagnostic device may set the first score and/or first grade as a first threshold value and/or first threshold grade. The processor of the diagnostic device may determine that the current risk of renal disease is high when the probability value and/or grade for the probability of occurrence of renal disease-related events within 5 years of the subject obtained from the second diagnostic model (1200) is equal to or greater than a predetermined first threshold value and/or first threshold grade. Also, in the above example, one threshold score and/or threshold grade was described as one. However, it is not limited thereto, and the threshold score and/or threshold grade may be set to a plurality or more. For example, the threshold score and/or threshold grade may include a first threshold value and/or first threshold grade corresponding to high risk of the existing biomarker and a second threshold value and/or second threshold grade corresponding to moderate risk of the existing biomarker.

Also, in one embodiment, the second diagnostic model (1200) may be trained based on training target data. Here, training of the second diagnostic model (1200) may include the meaning of fitting the second diagnostic model (1200). Also, the second diagnostic model (1200) may be constructed using a linear regression-based Cox proportional hazards model.

As an example, the training target data may include physical information of subjects of training target retinal images of the first diagnostic model (1100), follow-up observation results of renal disease events of the subjects for 5 years, and probability values of the first diagnostic model (1100) for the training target retinal images. Also, according to embodiments, subjects of training target retinal images of the first diagnostic model (1100) and subjects of training target data of the second diagnostic model (1200) may be the same or may be at least partially different. For example, among training target data of subjects of training target retinal images of the first diagnostic model (1100), training target data of subjects who already had renal disease events or are suffering from renal disease at a reference time point may be excluded from training of the second diagnostic model (1200). This is because, by training the second diagnostic model (1200) using training target data for normal persons who do not have renal disease events and do not suffer from renal disease, accuracy of the probability of occurrence of renal disease events within 5 years output from the second diagnostic model (1200) can be increased.

Also, in one embodiment, in the training process of the second diagnostic model (1200), coefficients of the covariates of the Cox proportional hazards model may be set by fitting the Cox proportional hazards model to the UK Biobank cohort. And, in the UK Biobank cohort, the survival probability for renal disease within 5 years may be 0.9980896. And, the second diagnostic model (1200) may be modeled as a failure probability within 5 years. Example 1 is described using the UK Biobank cohort as an example, but the description of the present specification is not limited thereto, and the second diagnostic model may be fitted or calibrated based on other cohorts other than the UK Biobank cohort.

Also, in one embodiment, the second diagnostic model (1200) may include as variables age, sex, presence or absence of hypertension, presence or absence of diabetes, and a probability value for the current risk of renal disease output from the first diagnostic model (1100). Since these variables can be obtained from subjects by non-invasive methods, they can be used in the second diagnostic model (1200).

For example, the second diagnostic model (1200) may obtain a probability value for the probability of occurrence of renal disease-related events within 5 years of a subject based on Equation 1 below. For example, the Cox proportional hazards model of the second diagnostic model (1200) may be constructed based on Equation 1 and Equation 2.

[Equation 1]

$$\text{Predicted risk} = 1 - SP^{\wedge}(\text{Exp}[LP])$$

Here, Predicted risk is a probability value for the probability of occurrence of renal disease-related events within 5 years, and SP (Survival Probability) is a survival probability for renal disease within 5 years, and for example, in the UK Biobank example described above, it may be 0.9980896. And, LP (Linear Predictor) is described in Equation 2 below.

[Equation 2]

$$LP = a1*RPS*100 + a2*Age + a3*Female + a4*Hypertension + a5*Diabetes$$

Here, RPS (Retinal photograph-based Prediction Score) is a probability value for the current risk of renal disease output from the first diagnostic model (1100), Age is age, and Female represents the sex of the subject. For example, Female may be an indicator function that reflects a value of 1 when the subject is female and reflects a value of 0 when the subject is male. And, Hypertension may reflect a value of 1 when the subject has hypertension and may reflect a value of 0 when the subject does not have hypertension. For example, in the case of a user taking antihypertensive medication, it may be set as having hypertension. Also, Diabetes may reflect a value of 1 when the subject is a diabetic patient (or in pre-diabetic stage) and may reflect a value of 0 when the subject is not a diabetic patient (or in pre-diabetic stage).

And, a1 to a5 are coefficients of covariates of each variable, and for example, through fitting of the second diagnostic model (1200), a1 to a5 may be set as 0.0546351, 0.0357466, -0.3317242, -0.1207903, and 0.4679712, respectively. Also, the processor of the diagnostic device may present the risk of renal disease within 3 years, 5 years, or 10 years based on Equation 1 and Equation 2 (or by applying Equation 1 and Equation 2). For example, the processor of the diagnostic device may add or replace race, smoking status, cholesterol level, etc. as covariates in Equation 2, and set coefficients of each covariate through fitting of the second diagnostic model (1200).

Also, the processor of the diagnostic device may obtain a probability value for the probability of occurrence of renal disease-related events within 5 years from the second diagnostic model (1200), and apply a predetermined cut-off value to the probability value to obtain a grade corresponding to the probability value. Of course, in some cases, a predetermined cut-off value may be applied in advance to the second diagnostic model (1200) to obtain a grade corresponding to the probability value from the second diagnostic model (1200). In this case, there may be one or more cut-off values. For example, when there is one cut-off value, there may be two grades corresponding to the probability value, and when there are two cut-off values, there may be three grades corresponding to the probability value. The description of the present specification can be applied to various numbers of cut-off values and various cut-off values.

In one embodiment, the processor of the diagnostic device may set a predetermined cut-off value based on population distribution (that is, incidence rate in each group) of each group divided into a plurality of grades according to follow-up observation results of a predetermined population. For example, for a predetermined population, a cut-off value may be determined based on results of follow-up observation. For example, based on all follow-up observation subjects, when follow-up observation results of 0~n1% as low-risk group, n1~n2% as moderate risk group, n2~n3% as high-risk group, and n3-100% as highest risk group are obtained, n1%, n2%, and n3% may each be set as cut-off values. Also, even if n1%, n2%, and n3% are not each cut-off values, cut-off values may be set so that the population distribution in each risk group of renal disease diagnostic information becomes similar to the follow-up observation results of a predetermined population.

As an example, when the predetermined population is the UK Biobank cohort and the Korean Diabetic cohort, according to the population distribution of the low-risk group, moderate risk group, high-risk group, and highest risk group in the UK Biobank cohort and the Korean diabetic cohort, cut-off values may be set so that the population distribution in each risk group of renal disease diagnostic information becomes similar to the population distribution in each risk group of the UK Biobank cohort and the Korean diabetic cohort.

Also, the processor of the diagnostic device may set a cut-off value based on the incidence rate in renal disease biomarkers. For example, in the case of eGFR, based on the eGFR level, the risk of renal disease can be classified as low risk (90 mL/min/1.73m$^2$ or more), moderate risk (60-89 mL/min/1.73m$^2$), moderate-high risk (45-59 mL/min/1.73m$^2$), very-

high risk (30-44 mL/min/1.73m$^2$), and highest risk (29 mL/min/1.73m$^2$ or less). And, when the incidence rate at the eGFR level, that is, persons corresponding to low risk, moderate risk, moderate-high risk, very-high risk, and highest risk are x1%, x2%, x3%, x4%, and x5%, respectively, cut-off values may be set so that the ratio of persons included in each grade of renal disease diagnostic information output from the processor of the diagnostic device matches the x1%, x2%, x3%, x4%, and x5%. This may be applied not only to the eGFR level but also to biomarkers such as albuminuria level, cystatin C level, KDIGO grade, etc.

Also, the incidence rate according to the Kidney Failure Risk Equation and the incidence rate according to the Risk Prediction Equation may also be used for setting the cut-off value described above.

Also, the cut-off value may be a value optimized to best detect groups corresponding to high risk of each biomarker.

As a specific example, the processor of the diagnostic device may set a cut-off value using the ratio of high-risk group and low-risk group classified by a 5-year kidney risk calculator according to race or the 5-year kidney risk calculation method of applicable clinical guidelines. Accordingly, performance of predicting the high-risk group of renal disease diagnostic information may be improved. For example, in order to maximize the performance of renal disease diagnostic information for diagnosing the high-risk group in the eGFR level, the cut-off value of renal disease diagnostic information may be set so that the ratio of all subjects corresponding to the low-risk group of renal disease diagnostic information and the ratio of all subjects corresponding to the high-risk group become similar to the ratio of subjects corresponding to the low-risk group of the eGFR level and the ratio of subjects corresponding to the high-risk group. For example, the processor of the diagnostic device may set the cut-off value of renal disease diagnostic information so that the ratio of all subjects corresponding to the low-risk group of renal disease diagnostic information and the ratio of all subjects corresponding to the high-risk group are included within a predetermined range of the ratio of subjects corresponding to the low-risk group of the eGFR level and the ratio of subjects corresponding to the high-risk group. In this case, the processor of the diagnostic device can accurately determine subjects corresponding to the low-risk group and high-risk group of the eGFR level.

Also, the biomarker used for training the first diagnostic model (1100) and the biomarker used for setting the cut-off value of the second diagnostic model (1200) may be the same or may be different.

Also, when a cut-off value based on each biomarker is set, the grade output as renal disease diagnostic information from the processor of the diagnostic device may be similar to the actual result of the biomarker for which the cut-off value was set. For example, when a cut-off value according to the biomarker of the eGFR level is applied to the probability value for the probability of occurrence of renal disease-related events within 5 years of a subject output from the second diagnostic model (1200), and the grade of the subject is output from the processor of the diagnostic device, the grade of the subject output from the processor of the diagnostic device and the grade determined by the subject actually performing an examination according to eGFR may be matched. When an actual examination is performed according to eGFR, albuminuria level, and cystatin C, inconveniences such as the subject undergoing a blood test or urine test may occur. However, in the case of the method for diagnosing renal disease according to the present specification, since renal disease diagnostic information with high accuracy is obtained using only the retinal image of the subject, user convenience may be improved.

Also, in one embodiment, when renal disease diagnostic information is applied to scores and/or grades of existing biomarkers, the incidence rate of renal disease-related events according to the scores and/or grades of existing biomarkers may be classified or stratified according to each group (for example, low-risk group, moderate risk group, high-risk group, and highest risk group) of renal disease diagnostic information. And, the cut-off of renal disease diagnostic information may be set so that when renal disease diagnostic information is applied to scores and/or grades of existing biomarkers, the incidence rate of renal disease-related events according to the scores and/or grades of existing biomarkers is clearly stratified according to each group of renal disease diagnostic information.

Hereinafter, examples of the method for diagnosing renal disease according to the present specification will be described in detail.

2.1.3. Example 1

Example 1 describes experimental results of the method for diagnosing renal disease according to the present specification using clinical data and retinal images from the UK Biobank cohort and the Korean diabetic cohort.

First, in Example 1, the first diagnostic model (1100) may be trained based on 158,216 (79,108 persons) retinal images and clinical data from a Korean health screening center (whether the eGFR level is 60 mL/min/1.73 m$^2$ or less for each retinal image and/or whether albuminuria is present in the urine of the subject).

Also, in Example 1, the second diagnostic model (1200) may be trained (or fitted) using the UK Biobank cohort (30,477) and the Korean diabetic cohort (5,014). Here, data of participants with chronic kidney disease, participants with eGFR levels less than 90 mL/min/1.73 m$^2$, or participants in whom albuminuria was detected (or when the albuminuria level is 30 mg/Cr or more) may be excluded from training of the second diagnostic model (1200). In this case, the processor of the diagnostic device may provide a probability value and/or grade for the probability of occurrence of renal disease-related events within 5 years with high accuracy for normal persons who do not currently have renal disease.

In addition, the content described in FIG. 20 may be applied to the first diagnostic model (1100) and the second diagnostic model (1200) in Example 1.

Hereinafter, results according to Example 1 will be described. In the following drawings, the Reti-CKD score represents a probability value for the probability of occurrence of renal disease-related events within 5 years as renal disease diagnostic information, and each grade of the Reti-CKD score may represent a grade corresponding to each probability value as renal disease diagnostic information.

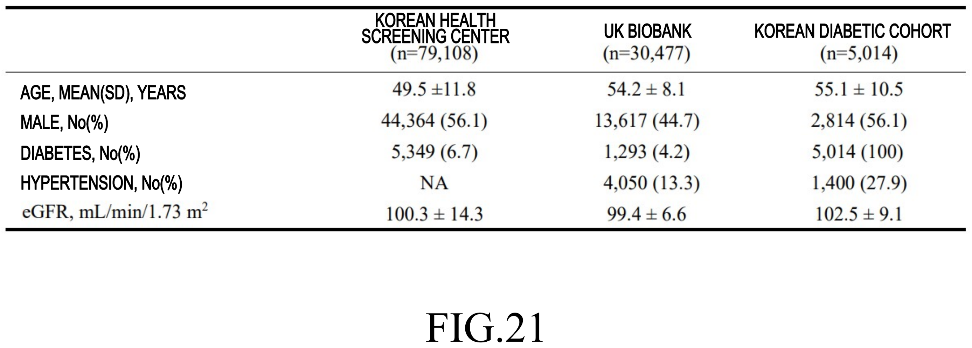

FIG. 21 shows clinical characteristics of subjects according to renal disease diagnostic information according to Example 1.

Referring to FIG. 21, data from the Korean Health screening data used for training the first diagnostic model (1100) is for 79,108 persons, the average age is 49.5 years (standard deviation (SD): 11.8), and the average eGFR level may be 100.3 (standard deviation (SD): 14.3) mL/min/1.73 $m^2$. Also, data from the UK Biobank cohort used for training the second diagnostic model (1200) is for 30,477 participants, and 720 (2.4%) of the participants may be diagnosed with chronic kidney disease during an average follow-up period of 10.8 years (interquartile range (IQR): 10.7-11.0). Also, data from the Korean diabetic cohort used for training the second diagnostic model (1200) is for 5,014 participants, and 206 (4.1%) of the participants may be diagnosed with chronic kidney disease during an average follow-up period of 6.1 years (interquartile range (IQR): 4.0-8.4). Also, the average eGFR level of the UK Biobank cohort may be 99.4 (standard deviation (SD): 6.6) mL/min/1.73 $m^2$, and the average eGFR level of the Korean diabetic cohort may be 102.5 (standard deviation (SD): 9.1) mL/min/1.73 $m^2$.

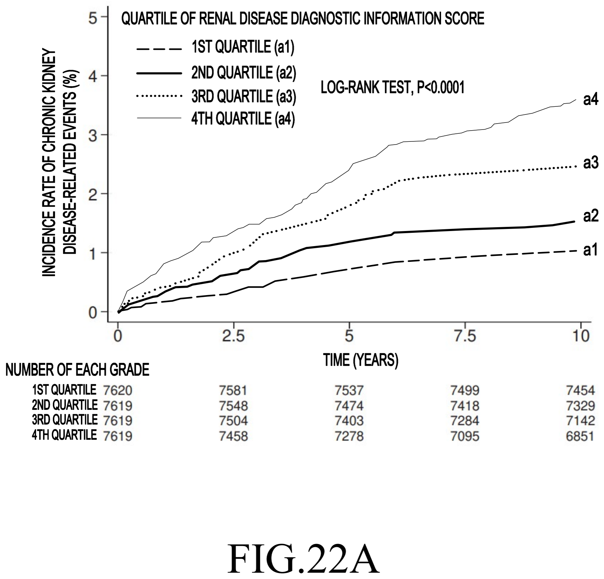

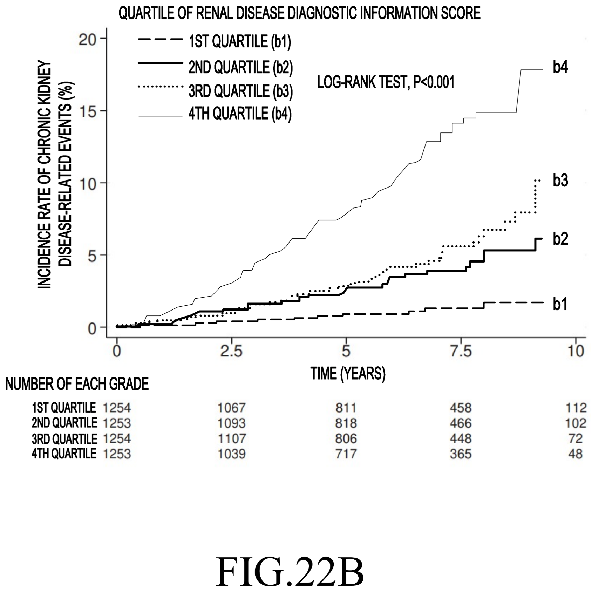

FIGS. 22A and 22B are diagrams for explaining the incidence rate of renal disease-related events according to renal disease diagnostic information according to Example 1.

Referring to FIGS. 22A and 22B, in the graphs of FIGS. 22A and 22B, the x-axis represents time (years) and the y-axis represents the incidence rate of chronic kidney disease-related events.

And, FIG. 22A shows a Kaplan-Meier curve representing results of performing Kaplan-Meier survival analysis on subjects of 4 groups according to 4 grades of renal disease diagnostic information in the UK Biobank cohort, and FIG. 22B may show a Kaplan-Meier curve representing results of performing Kaplan-Meier survival analysis on subjects of 4 groups according to 4 grades of renal disease diagnostic information in the Korean diabetic cohort. In the UK Biobank cohort of FIG. 22A, 321,317 person-years were examined during an average follow-up period of 10.8 years, and in the Korean diabetic cohort of FIG. 22B, 30,122 person-years were examined during an average follow-up period of 6.1 years.

As shown in FIGS. 22A and 22B, renal disease diagnostic information can clearly distinguish renal disease risk based on 4 groups in the UK Biobank cohort and Korean diabetic cohort. In FIGS. 22A and 22B, the Hazard Ratio (HR) of chronic kidney disease occurrence may show a dose-dependent association according to the 4 grades. Adjusted HRs per 1 SD increment of renal disease diagnostic information may be 1.34 (95% confidence interval: 1.27-1.41) in the UK Biobank cohort of FIG. 22A and 1.94 (95% confidence interval: 1.63-2.31) in the Korean diabetic cohort of FIG. 22B.

Also, renal disease diagnostic information can clearly distinguish renal disease risk with significant hazard ratios even in subgroups according to sex, age, presence or absence of hypertension, and presence or absence of diabetes. For example, in the UK Biobank cohort of FIG. 22A, hazard ratio trends for chronic kidney disease occurrence may be: Overall_1.52 (95% confidence interval: 1.42-1.63), Male_1.47 (95% confidence interval: 1.31-1.65), Female_1.51 (95% confidence interval: 1.36-1.67), Under 55_1.35 (95% confidence interval: 1.20-1.51), Over 55_1.46 (95% confidence interval: 1.29-1.65), No hypertension_1.59 (95% confidence interval: 1.47-1.74), Hypertension_1.32 (95% confidence interval: 1.12-1.56), No diabetes_1.50 (95% confidence interval: 1.39-1.61), Diabetes_1.35 (95% confidence interval: 1.07-1.72), eGFR over 100_1.53 (95% confidence interval: 1.34-1.73), eGFR 100 or less_1.41 (95% confidence interval: 1.30-1.53). Also, in the Korean diabetic cohort of (b), hazard ratio trends for chronic kidney disease occurrence may be: Overall_1.99 (95% confidence interval: 1.72-2.31), Male_2.17 (95% confidence interval: 1.73-2.70), Female_1.85 (95% confidence interval: 1.51-2.28), Under 55_1.75 (95% confidence interval: 1.28-2.39), Over 55_1.97 (95% confidence interval: 1.58-2.46), No hypertension_2.13 (95% confidence interval: 1.76-2.57), Hypertension_1.71 (95% confidence interval: 1.37-2.12), eGFR over 100_2.10 (95% confidence interval: 1.62-2.72), eGFR 100 or less_1.49 (95% confidence interval: 1.28-1.78).

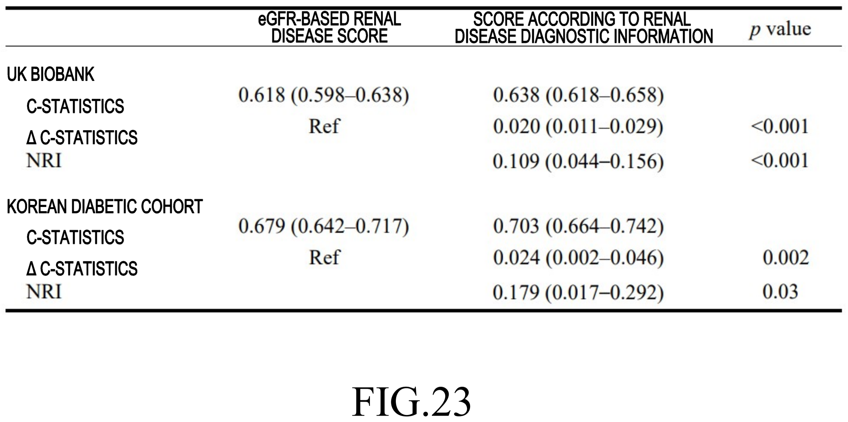

FIG. 23 is a diagram for explaining the performance of predicting the occurrence of renal disease-related events within 5 years of renal disease diagnostic information according to Example 1.

Referring to FIG. 23, the table of FIG. 23 compares the performance of the score according to renal disease diagnostic information of the present specification (Reti-CKD) and the eGFR-based renal disease score (eGFR-CKD score) in the UK Biobank cohort and Korean diabetic cohort. The eGFR-based renal disease score may be used as a biomarker for assessing the risk of existing renal disease.

In the table of FIG. 23, NRI (Net reclassification Index) may represent an index measuring how much better a new model is than a past model. For NRI, similar results may appear as 0.109 (95% confidence interval (CI): 0.44-0.156) in the UK Biobank cohort and 0.179 (95% confidence interval: 0.017-0.292) in the Korean diabetic cohort. And, when comparing the score according to renal disease diagnostic information with the eGFR-based renal disease score, the difference in C-statistics is 0.020 (95% confidence interval (CI): 0.011-0.029) in the UK Biobank cohort and 0.024 (95% confidence interval: 0.002-0.046) in the Korean diabetic cohort, and the score according to renal disease diagnostic information may appear significantly greater than the eGFR-based renal disease score. Accordingly, the score according to renal disease diagnostic information may show excellent performance with higher accuracy compared to the existing eGFR-based renal disease score.

Also, although not shown in the table of FIG. 23, when performing sensitivity analysis on all subjects without including only subjects with eGFR levels of 90 mL/min/1.73 m2 or more, when comparing the score according to renal disease diagnostic information with the eGFR-based renal disease score, the difference in C-statistics is 0.008 (95% confidence interval (CI): 0.001-0.016) in the UK Biobank cohort and 0.057 (95% confidence interval: 0.048-0.067) in the Korean diabetic cohort, and the score according to renal disease diagnostic information may appear significantly greater than the eGFR-based renal disease score. Accordingly, the score according to renal disease diagnostic information may show excellent performance with higher sensitivity compared to the existing eGFR-based renal disease score. Therefore, even if eGFR examination does not precede through blood tests (for example, even if it is not known whether the eGFR level is 90 mL/min/1.73 m2 or more), renal disease diagnostic information can better stratify the risk of future renal disease than eGFR for general populations and diabetic patients.

Also, although not shown in the table of FIG. 23, sensitivity analysis may be performed by dividing into a group with hypertension and/or diabetes and a group without hypertension and diabetes.

When comparing the score according to renal disease diagnostic information with the eGFR-based renal disease score in the group without hypertension and diabetes, the difference in C-statistics is 0.025 (95% confidence interval (CI): 0.002-0.048) in the UK Biobank cohort, and the score according to renal disease diagnostic information may appear significantly greater than the eGFR-based renal disease score. Accordingly, the score according to renal disease diagnostic information may show excellent performance with higher sensitivity than the existing eGFR-based renal disease score for persons with normal kidney function without hypertension and diabetes.

Also, in the group with hypertension and/or diabetes, when comparing the score according to renal disease diagnostic information with the eGFR-based renal disease score, the difference in C-statistics is 0.019 (95% confidence interval (CI): 0.008-0.030) in the UK Biobank cohort, and the score according to renal disease diagnostic information may appear significantly greater than the eGFR-based renal disease score. Accordingly, the score according to renal disease diagnostic information may show excellent performance with higher sensitivity than the existing eGFR-based renal disease score regardless of the presence or absence of hypertension and/or diabetes. Therefore, it can be seen that risk prediction is possible not only for application to hypertension and diabetic patient groups, which are the most important risk factors for renal disease, but also for renal diseases occurring due to problems with the kidney itself regardless of hypertension/diabetes.

2.1.4. Obtaining Information Regarding Renal Disease Risk According to Existing Biomarkers

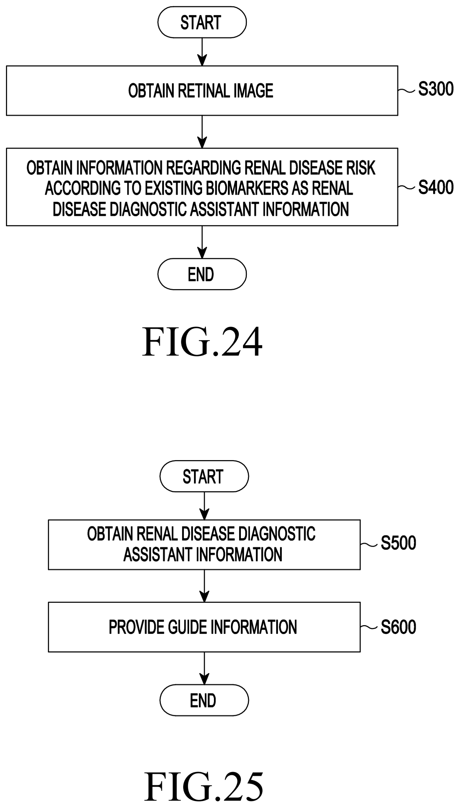

FIG. 24 is a diagram for explaining a method for diagnosing renal disease according to another embodiment.

Referring to FIG. 24, the method for diagnosing renal disease according to another embodiment may include obtaining a retinal image (S300) and obtaining information regarding renal disease risk according to existing biomarkers as renal disease diagnostic information (S400).

In step S300, the processor of the diagnostic device may obtain a retinal image. Since the content described in FIG. 19 and above can be applied to this, detailed description will be omitted.

Also, in step S400, the processor of the diagnostic device may obtain information regarding renal disease risk according to existing biomarkers as renal disease diagnostic information. The content described in the diagnostic model described above may be applied to step S400.

Here, information regarding renal disease risk according to existing biomarkers means renal disease risk calculated according to existing biomarkers, and the processor of the diagnostic device may obtain information regarding renal disease risk calculated according to existing biomarkers using retinal images. For example, according to step S400, the processor of the diagnostic device may obtain information regarding eGFR level and/or grade according to eGFR level, albuminuria level and/or grade according to albuminuria level, cystatin C level and/or grade according to cystatin C level using a diagnostic model based on retinal images, and output the obtained information, or output information regarding a final score and/or grade according to the final score based on the obtained information.

In one embodiment, the processor of the diagnostic device may obtain information regarding renal disease risk according to existing biomarkers as renal disease diagnostic information using a diagnostic model.

As an example, learning may be performed using retinal images and information regarding renal disease risk according to existing biomarkers labeled on the retinal images. For example, at least one of eGFR level (and/or grade accordingly), albuminuria level (and/or grade accordingly), or cystatin C level (and/or grade accordingly) may be labeled on each retinal image, and the diagnostic model may be learned using retinal images and corresponding labels. Also, the label labeled on the retinal image may include information regarding at least one of age, sex, race, smoking status, blood pressure (presence or absence of hypertension), and presence or absence of diabetes of the subject of the retinal image.

The processor of the diagnostic device may obtain information regarding renal disease risk according to existing biomarkers by inputting a retinal image of a subject to a learned single diagnostic model. For example, when the diagnostic model is learned based on eGFR levels, the processor of the diagnostic device may input a retinal image to the diagnostic model and obtain information regarding eGFR level and/or grades different from the eGFR level from the diagnostic model.

Also, according to the embodiment, the processor of the diagnostic device may input information regarding at least one of age, sex, race, smoking status, blood pressure (presence or absence of hypertension), presence or absence of diabetes, or cholesterol level of the subject together with the retinal image to the diagnostic model, and obtain information regarding eGFR level and/or grades different from the eGFR level from the diagnostic model.

Also, in one embodiment, the diagnostic model may be configured in parallel. For example, the description of the diagnostic models of 1.4.1.1 to 1.4.1.3 may be applied to the diagnostic model.

Specifically, the diagnostic model may include a plurality of diagnostic models. For example, the diagnostic model may include a first diagnostic model and a second diagnostic model. The first diagnostic model and the second diagnostic model may be learned using information regarding renal disease risk according to different biomarkers. For example, the first diagnostic model may be learned using retinal images labeled with eGFR levels (and/or grades accordingly), and the second diagnostic model may be learned using retinal images labeled with albuminuria levels (and/or grades accordingly). Of course, without being limited thereto, the diagnostic model may include additional diagnostic models such as third/fourth diagnostic models together with the first/second diagnostic models. Also, the label labeled on the retinal image may include information regarding at least one of age, sex, race, smoking status, blood pressure (presence or absence of hypertension), and presence or absence of diabetes of the subject of the retinal image.

Also, the processor of the diagnostic device may obtain information regarding renal disease risk according to different biomarkers by inputting a retinal image of a subject to the first diagnostic model and the second diagnostic model. For example, the processor of the diagnostic device may obtain the eGFR level (and/or grade accordingly) of the subject from the first diagnostic model, obtain the albuminuria level (and/or grade accordingly) of the subject from the second diagnostic model, and output the obtained information. Also, the processor of the diagnostic device may output information regarding a final score and/or grade according to the final score based on information obtained from each diagnostic model. For example, information regarding a final score and/or grade according to the final score may be obtained and output based on the eGFR level (and/or grade accordingly) of the subject obtained from the first diagnostic model and the albuminuria level (and/or grade accordingly) of the subject obtained from the second diagnostic model. Accordingly, the processor of the diagnostic device may non-invasively obtain information regarding renal disease risk according to existing biomarkers as renal disease diagnostic information using retinal images without using invasive methods such as blood tests.

Also, in one embodiment, the diagnostic model may be configured in series. For example, the description of the diagnostic models of 1.4.2.1, 1.4.2.2, and 2.1.2 may be applied to the diagnostic model.

As an example, the diagnostic model may include a first diagnostic model and a second diagnostic model as shown in FIG. 20. For example, the processor of the diagnostic device may input a retinal image of a subject to the first diagnostic model, and obtain from the first diagnostic model a probability value that the subject is currently at high risk for renal disease (the probability that the eGFR level of the subject is 60 mL/min/1.73 m2 or less and/or the probability that albuminuria is present in the urine of the current subject). Also, the processor of the diagnostic device may input the output value of the first diagnostic model and physical information of the subject (at least one of age, sex, race, smoking status, blood pressure (for example, blood pressure level, presence or absence of hypertension), presence or absence of diabetes (or blood sugar level), cholesterol level). At this time, cut-off values based on various biomarkers such as eGFR level, albuminuria level, and cystatin C level may be set in the second diagnostic model. For example, as a cut-off value of the second diagnostic model, a cut-off value set to be similar to the ratio of persons corresponding to 45-59 mL/min/1.73m$^2$, which is the standard for the moderate-high risk group of eGFR level, and/or a cut-off value set to be similar to the ratio of persons corresponding to 30-300 mg/g, which is the standard for the moderate risk group of albuminuria level, may be set. Accordingly, renal disease diagnostic information can predict the moderate-high risk group (or moderate risk group) according to various biomarkers with high accuracy.

2.1.5. Providing Guide Information Regarding Renal Disease Diagnostic Information

FIG. 25 is a diagram for explaining a method for providing guide information regarding renal disease diagnostic information according to one embodiment.

Referring to FIG. 25, the method for providing guide information according to one embodiment may include obtaining renal disease diagnostic information (S500) and providing guide information regarding diagnostic information (S600).

In step S500, the processor of the diagnostic device may obtain diagnostic information. Since the description above may be applied to step S500, detailed description will be omitted.

Also, in step S600, the processor of the diagnostic device may provide guide information regarding diagnostic information. In steps S500 and S600, description is focused on renal disease for convenience of explanation, but is not limited thereto, and the guide information of the present specification may also be applied to various diseases such as ophthalmic disease, cardiovascular disease, etc., of course. Accordingly, in steps S500 and S600, description is focused on guide information according to renal diagnostic information.

In one embodiment, guide information may mean information regarding medical/non-medical treatment recommended to a subject according to renal disease diagnostic information. As an example, guide information may include prescription information, action information, and management information.

Prescription information may mean information regarding drugs (for example, prescription drugs) recommended to a subject in order to maintain or improve renal disease risk according to renal disease diagnostic information. Also, prescription information may include information regarding prescribed drugs, dosing time, and dosage. For example, prescription information may include information regarding prescription of one or more of ACE inhibitors (Angiotensin-Converting Enzyme Inhibitors) (for example, Lisinopril, Enalapril), Hydrochlorothiazide), cholesterol-lowering agents (for example, statin drugs, Atorvastatin, Rosuvastatin), phosphate binders (for example, Calcitriol, Sevelamer), and anticoagulants (for example, Warfarin). Also, prescription information may include SGLT2 inhibitors (Sodium-Glucose Co-Transporter 2 Inhibitors) used in diabetes prescriptions as combination drugs (for example, Dapagliflozin, Empagliflozin, Canagliflozin), ARBs (Angiotensin II Receptor Blockers) used in hypertension prescriptions as combination drugs (for example, Losartan, Valsartan), GLP1 (Glucagon-Like Peptide-1) agonists used in diabetes and obesity prescriptions as combination drugs, and/or Diuretics (for example, Furosemide).

Also, the processor of the diagnostic device may provide prescription information in consideration of drugs that require caution when administered to patients with renal disease. For example, drugs that require caution when administered to patients with the disease include analgesics and antipyretics (nonsteroidal anti-inflammatory drugs (NSAIDs), high-dose aspirin), antibiotics (Aminoglycosides, Amphotericin B, Cephalosporins, Penicillins, Beta-lactamase inhibitors, Quinolones, Rifampin, Sulfonamides, Vancomycin), antivirals (Acyclovir, Adefovir, Ganciclovir, Atazanavir, Indinavir, Tenofovir), Bisphosphonates (Pamidronate, Zoledronic acid), Calcineurin inhibitors (Cyclosporine, Tacrolimus), anticancer drugs (Alkylating agents, Cisplatin, Methotrexate, Mitomycin, Interferon-alpha, Proteasome inhibitors, Vascular endothelial growth factor (VEGF) inhibitors, Checkpoint inhibitors), iodinated contrast agents used in CT and angiography (Iodine contrast agent), diuretics (Loop diuretic, Thiazides, Triamterene), Proton pump inhibitors (Dexlansoprazole, Esomeprazole, Lansoprazole, Omeprazole, Pantoprazole, Rabeprazole), other drugs (Allopurinol, Gold sodium thiomalate, Lithium, Quinine, Sodium phosphate), herbal medicines (Aristolochic acid, Cats claw, Licorice root).

Also, action information may mean information regarding future actions recommended to a subject in order to maintain or improve renal disease risk according to renal disease diagnostic information. For example, additional examination information may include information regarding secondary diagnosis or medical treatment for the subject. As an example, additional examination information may include additional required tests, information regarding hospitals/medical staff where additional tests are possible, and information regarding recommended procedures/surgeries.

Also, management information may include information regarding non-medical treatment recommended to a subject in order to maintain or improve renal disease risk according to renal disease diagnostic information. For example, management information may include lifestyle information for reducing renal disease risk, dietary information, exercise information, information regarding non-prescription drugs such as nutritional supplements, etc.

Also, in one embodiment, the diagnostic device may be linked with an external monitoring device. Here, the monitoring device may mean a device that monitors the lifestyle or behavior of a subject. For example, the monitoring device may include a portable device, wearable device, wellness measurement device, etc. Also, the monitoring device may be the client device described above. For example, the monitoring device may include an imaging unit and may photograph the inside and outside of the eye through the imaging unit to obtain images of the inside and outside of the eye.

And, the monitoring device may monitor various information such as the subject's activity level, exercise method, exercise time, food intake, intake amount, health supplement intake information, sleep time, sleep habits, heart rate, blood pressure, blood sugar level, body water content, oxygen level, body temperature, oxygen saturation, pulse wave, hospital visit status, whether examination was received, whether procedure/surgery was received, ocular images, eGFR level, albuminuria level, cystatin C level, etc.

The processor of the diagnostic device may perform wired or wireless communication with the monitoring device through a communication module.

The processor of the diagnostic device may provide guide information to the monitoring device. And, the monitoring device may provide various information to the subject based on guide information and monitored information. For example, the monitoring device may obtain management information (for example, lifestyle information, dietary information, exercise information) as guide information from the diagnostic device, compare the management information with monitored information, determine whether the monitored information matches the management information, and provide determination results and/or additional information accordingly.

For example, when monitored exercise time is less than exercise time in management information, the monitoring device may provide information to the subject to exercise according to the management information. Also, when food intake among monitored information corresponds to food intake in management information, the monitoring device may provide information to the subject that food intake is being done well according to the management information.

Also, the processor of the diagnostic device may obtain monitored information from the monitoring device. The processor of the diagnostic device may provide various information to the subject based on guide information and monitored information. For example, the processor of the diagnostic device may compare monitored information with guide information, determine whether the monitored information matches management information, and provide determination results and/or additional information accordingly. The example of the monitoring device described above may be applied to the operation of the processor of the diagnostic device.

Also, the processor of the diagnostic device may generate guide information by reflecting monitoring information received from the monitoring device. For example, the processor of the diagnostic device may obtain status information of the subject (exercise status, lifestyle status, dietary habit status, etc.) based on monitoring information, and modify guide information determined as renal disease diagnostic information based on the status information of the subject to suit the subject.

In one embodiment, the processor of the diagnostic device may provide guide information using a predetermined database. For example, the diagnostic device may include a database matching scores and/or grades of renal disease diagnostic information with guide information. For example, when renal disease diagnostic information is expressed in 3 grades, guide information matching low-risk grade (for example, prescription information-none, action information-information regarding next treatment time, management information-provision of dietary information, provision of exercise information), guide information matching moderate risk grade (for example, prescription information-none, action information-provision of additional test information, management information-provision of dietary information, provision of exercise information, provision of non-prescription drug information), and guide information matching high-risk grade (for example, prescription information-provision of statin prescription information, action information-additional test information, provision of recommended procedure/surgery information, management information-provision of dietary information, provision of exercise information, provision of non-prescription drug information) may be included in the database. The processor of the diagnostic device may provide guide information matching renal disease diagnostic information based on the database.

Also, in another embodiment, the processor of the diagnostic device may provide guide information using a machine learning model. For example, the diagnostic device may include a guide information model based on a machine learning model or neural network model. The guide information model may be learned based on scores and/or grades of renal disease diagnostic information and guide information. Also, additionally, the guide information model may also be learned for physical information of various subjects (for example, at least one of height, weight, sex, race, smoking status, blood pressure (for example, blood pressure level, presence or absence of hypertension), presence or absence of diabetes (or blood sugar level), cholesterol level). Accordingly, the processor of the diagnostic device may input scores and/or grades of renal disease diagnostic information and physical information of the subject to the guide information model to obtain guide information for the subject. Also, according to embodiments, the guide information model may be included in the diagnostic model or may be configured independently from the diagnostic model.

2.1.6. Providing Guide Information Using Existing Prescription Information and Renal Disease Assitant Information

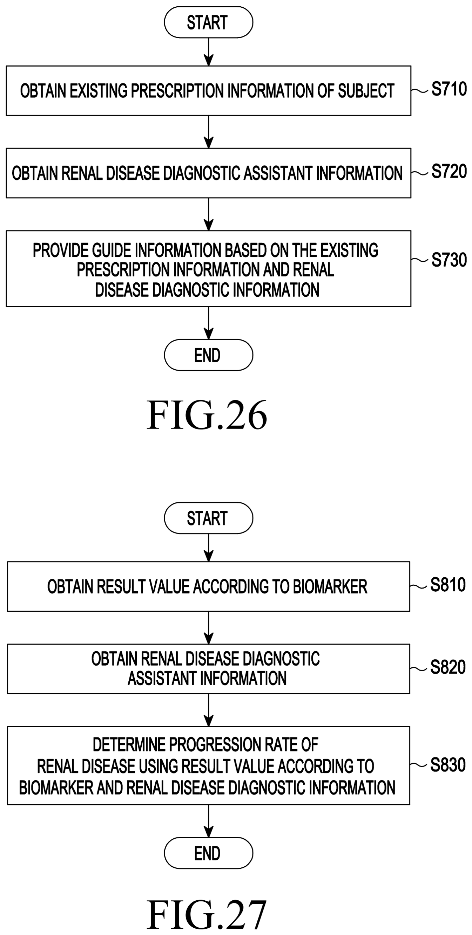

FIG. 26 is a diagram for explaining a method for providing guide information using existing prescription information and renal disease diagnostic information according to one embodiment.

Referring to FIG. 26, the method for providing guide information according to one embodiment may include obtaining existing prescription information of a subject (S710), obtaining renal disease diagnostic information (S720), and providing guide information based on the existing prescription information and renal disease diagnostic information (S730).

In some cases, renal disease often progresses together with underlying diseases such as diabetes, hypertension, and/or obesity. And, some of the prescription drugs for diabetes, hypertension, and/or obesity often have therapeutic effects on renal disease as well. For example, SGLT2 inhibitors may be combination drugs that are prescription drugs for diabetes but also have therapeutic effects on renal disease, and ARBs may be combination drugs that are prescription drugs for hypertension but also have therapeutic effects on renal disease. Also, GLP1 (Glucagon-Like Peptide-1) agonists may be combination therapies that are prescription drugs for diabetes and/or obesity but also have therapeutic effects on renal disease.

If a subject is taking such combination drugs, it may be necessary to provide prescription information to the subject excluding drugs for which separate drug prescription for renal disease is unnecessary according to the subject's renal disease risk, or drugs that reduce the treatment effect on the subject when taken together with combination drugs. For example, there are research results that combination prescription of ARB, a hypertension prescription drug, and ACE inhibitor, a renal disease prescription drug, is not recommended. In this way, when providing guide information, especially prescription information, for a subject, accurate prescription information can be provided only by considering drugs the subject is already taking.

Accordingly, the processor of the diagnostic device may provide guide information, especially prescription information, based on the subject's existing prescription information and renal disease diagnostic information.

According to step S710, the processor of the diagnostic device may obtain existing prescription information of the subject. For example, the processor of the diagnostic device may obtain existing prescription information from an external device or an input module of the diagnostic device. Also, the processor of the diagnostic device may obtain information regarding underlying diseases of the subject (for example, presence or absence of hypertension, blood pressure level, presence or absence of diabetes, blood sugar level, presence or absence of obesity, BMI level, cholesterol level, etc.) instead of or together with existing prescription information.

Also, in step S720, the processor of the diagnostic device may obtain renal disease diagnostic information. Here, renal disease diagnostic information may mean renal disease diagnostic information based on a retinal image of the same subject as the subject of the existing prescription information (and/or information regarding underlying diseases) obtained in step S710. Since the description above may be applied to step S720, detailed description will be omitted.

Also, in step S730, the processor of the diagnostic device may provide guide information based on existing prescription information and renal disease diagnostic information.

The processor of the diagnostic device may provide guide information based on renal disease diagnostic information as in 2.1.5 described above. However, in step S730, guide information may be provided in consideration of not only renal disease diagnostic information but also existing prescription information (and/or information regarding underlying diseases).

In one embodiment, when the score and/or grade of renal disease diagnostic information corresponds to a low-risk group, the processor of the diagnostic device may not provide prescription information among guide information. And, when the subject receives a prescription for treating underlying disease through existing prescription information (and/or information regarding underlying diseases) or the risk of underlying disease is high, the processor of the diagnostic device may provide information helpful for treatment or management of the underlying disease when providing action information and/or management information.

Also, when the score and/or grade of renal disease diagnostic information corresponds to a moderate risk group and/or high-risk group, the processor of the diagnostic device may provide prescription information among guide information. If existing prescription information includes combination drugs that can treat both underlying disease and renal disease such as SGLT2 inhibitors, ARBs, and GLP1 agonists, prescription information may be provided in consideration of the risk of renal disease diagnostic information. For example, when the risk of renal disease diagnostic assistance is moderate risk, the processor of the diagnostic device may provide prescription information to maintain taking existing combination drugs without prescribing new renal disease treatments. Also, when the risk of renal disease diagnostic assistance is high risk, the processor of the diagnostic device may provide prescription information regarding renal disease treatments that can be taken together with combination drugs in existing prescription information.

Also, when existing prescription information does not include treatments for underlying disease such as combination drugs or the risk of underlying disease is low and the risk of renal disease diagnostic assistance is moderate risk or high risk, the processor of the diagnostic device may provide prescription information regarding renal disease treatments (or combination drugs).

However, this example is only one example among various embodiments of the present specification. Without being limited to this example, the processor of the diagnostic device may provide guide information using a predetermined database and/or guide information model, as described in 2.1.5. In this case, guide information according to existing prescription information and renal disease diagnostic information may be matched and stored in the database, or the guide information model may be learned according to guide information according to existing prescription information and renal disease diagnostic information. Accordingly, the processor of the diagnostic device may obtain guide information from the database using existing prescription information and renal disease diagnostic information, or may obtain guide information by inputting existing prescription information (or existing prescription information and renal disease diagnostic information) to the guide information model.

2.1.7. Predicting Renal Disease Progression Rate Using Renal Disease Diagnostic Information

FIG. 27 is a diagram for explaining a method for predicting renal disease progression rate using renal disease diagnostic information according to one embodiment.

Referring to FIG. 27, the method for predicting renal disease progression rate according to one embodiment may include obtaining a result value according to a biomarker (S810), obtaining renal disease diagnostic information (S820), and determining the progression rate of renal disease using the result value according to the biomarker and renal disease diagnostic information (S830).

In step S810, the processor of the diagnostic device may obtain a result value according to a biomarker. For example, the processor of the diagnostic device may obtain a score and/or grade of a biomarker from an external device or an input module of the diagnostic device. Here, the biomarker may include the eGFR level (and/or grade accordingly), albuminuria level (and/or grade accordingly), or cystatin C level (and/or grade accordingly) described above. In addition, the biomarker may include other renal disease biomarkers or risk assessment tools for renal disease.

Also, in step S820, the processor of the diagnostic device may obtain renal disease diagnostic information. Here, renal disease diagnostic information may mean renal disease diagnostic information based on a retinal image of the same subject as the subject of the result value according to the biomarker obtained in step S810. Since the description above may be applied to step S820, detailed description will be omitted.

Also, in step S830, the progression rate of renal disease may be determined using the result value according to the biomarker and renal disease diagnostic information.

Specifically, even if the result value according to the biomarker is the same, the progression rate of renal disease may be different. For example, even if the eGFR level is 50 mL/min/1.73m$^2$, which is a moderate-high risk group, the score of renal disease diagnostic information may be different. However, even if the eGFR level is the same, a person with a high score of renal disease diagnostic information may have a faster progression rate of renal disease than a person with a low score of renal disease diagnostic information.

This will be described in detail through Example 2.

2.1.7.1. Example 2

Example 2 is for predicting the renal disease progression rate using KDIGO grade and renal disease diagnostic information.

In Example 2, data of 5,346 diabetic patients from 2 tertiary hospitals in Korea may be used. This may include data of persons with eGFR <90 ml/min/1.73m2 or albuminuria. And, data of persons with missing information regarding retinal images, serum creatinine, or albuminuria may be excluded.

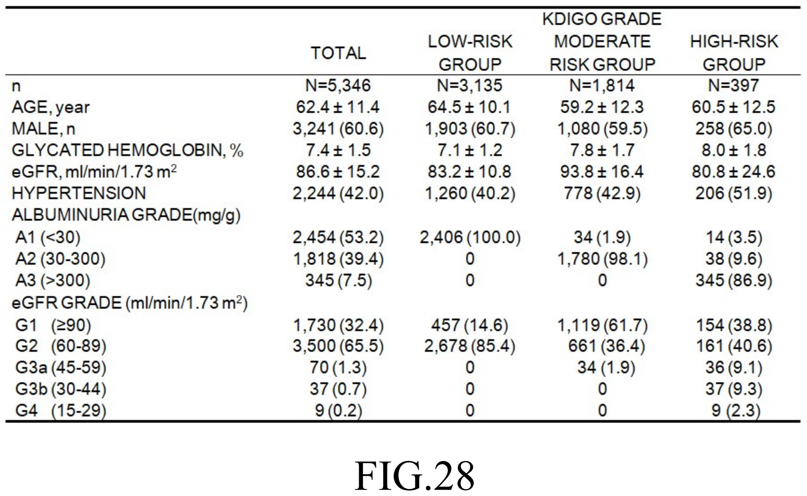

FIG. 28 is a diagram for explaining clinical characteristics of subjects according to Example 2.

Referring to FIG. 28, the KDIGO grade may be classified into 3 grades (low-risk group, moderate risk group, high-risk group) in which eGFR level (or grade) and albuminuria level (or grade) are considered. In the table of FIG. 28, based on albuminuria level, the risk of renal disease may be classified into low-risk group (A1, less than 30 mg/g), moderate risk group (A2, 30-300 mg/g), and high-risk group (A3, 300 mg/g or more). And, based on eGFR level, the risk of renal disease may be classified into low-risk group (G1, 90 mL/min/1.73m$^2$ or more), moderate risk group (G2, 60-89 mL/min/1.73m$^2$), moderate-high risk group (G3a, 45-59 mL/min/1.73m$^2$), very-high risk group (G3b, 30-44 mL/min/1.73m$^2$), and highest risk group (G4, 29 mL/min/1.73m$^2$ or less).

And, in the KIDGO grade, the low-risk group includes 3,135 persons including low-risk group according to albuminuria level, low-risk group according to eGFR level, and part of moderate risk group, the moderate risk group includes 1,814 persons including low-risk group and moderate risk group according to albuminuria level, low-risk group, moderate risk group, and part of moderate-high risk group according to eGFR level, and the high-risk group may include 397 persons including all risk groups according to albuminuria level and part of all risk groups according to eGFR level.

And, the average age of subjects may be 62.4 (+/- 11.4) years old and 60.6% may be male. The average eGFR of subjects is 86.6 (+/- 15.3 mL/min per 1.73 m2) and albuminuria may appear in 46.9%. During a follow-up period of 5.0 years (interquartile range: 2.5-7.8), renal disease-related events may occur in 1,379 (25.8%) subjects.

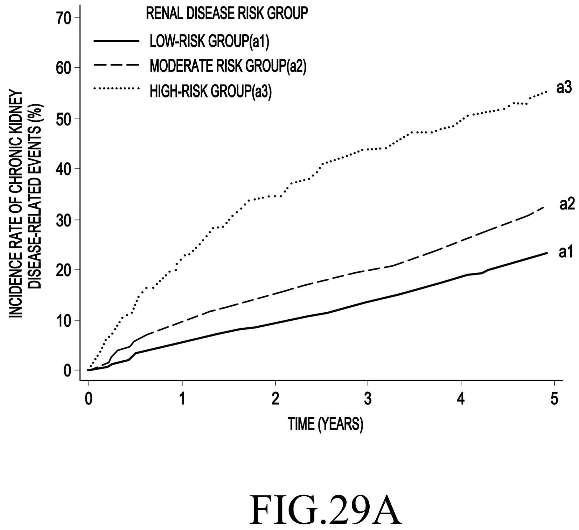

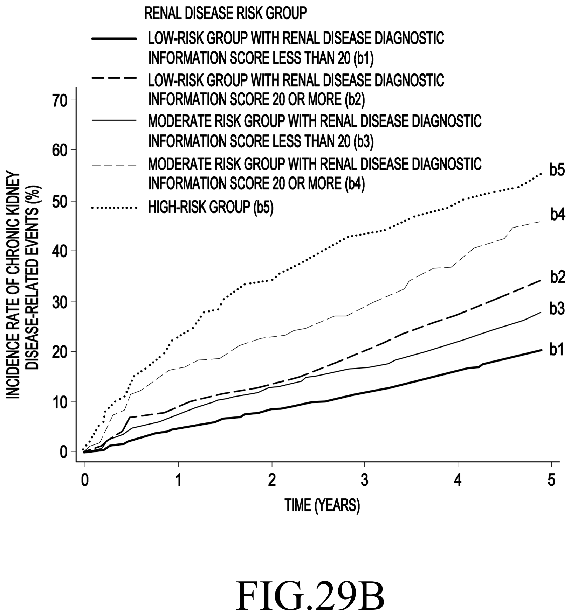

FIGS. 29A and 29B are diagrams for explaining the incidence rate of renal disease-related events according to KDIGO grade and renal disease diagnostic information according to Example 2.

Referring to FIGS. 29A and 29B, in the graphs of FIGS. 29A and 29B, the x-axis represents time (years) and the y-axis represents the incidence rate of chronic kidney disease-related events.

The graph of FIG. 29A shows the incidence rate of renal disease-related events according to KDIGO grade (low-risk group (a1), moderate risk group (a2), high-risk group (a3)), and the higher the high-risk group (a3), the higher the incidence rate of renal-related events may be.

The graph of FIG. 29B shows the incidence rate of renal disease-related events when scores according to renal disease diagnostic information are reflected in KDIGO grade.

Group 1 (b1) is a group of subjects with scores according to renal disease diagnostic information less than 20 among the low-risk group of KDIGO grade, Group 2 (b2) is a group of subjects with scores according to renal disease diagnostic information of 20 or more among the low-risk group of KDIGO grade, Group 3 (b3) is a group of subjects with scores according to renal disease diagnostic information less than 20 among the moderate risk group of KDIGO grade, Group 4 (b4) is a group of subjects with scores according to renal disease diagnostic information of 20 or more among the moderate risk group of KDIGO grade, and Group 5 (b1) may represent a group of the high-risk group of KDIGO grade.

Specifically, the incidence rate of renal disease-related events of the low-risk group (a1) in the graph of FIG. 29A may be classified or stratified like the incidence rates of renal disease-related events of Group 1 (b1) and Group 2 (b2) of (b). Here, the incidence rate of renal disease-related events of Group 1 (b1) of (b) is lower than the incidence rate of renal disease-related events of the low-risk group (a1) in the graph of FIG. 29A, and the incidence rate of renal disease-related events of Group 2 (b2) in the graph of FIG. 29B may be higher than the incidence rate of renal disease-related events of the low-risk group (a1) in the graph of FIG. 29A. That is, even if subjects equally correspond to the low-risk group of KDIGO grade, the incidence rate of renal disease-related events may be classified or stratified according to the score of renal disease diagnostic information. In other words, even if subjects equally correspond to the low-risk group of KDIGO grade, subjects with high scores of renal disease diagnostic information may have a higher probability of renal disease-related events occurring than subjects with low scores of renal disease diagnostic information. This may mean that even if subjects equally correspond to the low-risk group of KDIGO grade, when the score of renal disease diagnostic information is high, the renal disease progression rate may be faster than when the score of renal disease diagnostic information is low.

Also, the incidence rate of renal disease-related events of the moderate risk group (a2) in the graph of FIG. 29A may be classified or stratified like the incidence rates of renal disease-related events of Group 3 (b3) and Group 4 (b4) in the graph of FIG. 29B. Here, the incidence rate of renal disease-related events of Group 3 (b3) in the graph of FIG. 29B is lower than the incidence rate of renal disease-related events of the moderate risk group (a2) in the graph of FIG. 29A, and the incidence rate of renal disease-related events of Group 4 (b4) in the graph of FIG. 29B may be higher than the incidence rate of renal disease-related events of the moderate risk group (a2) in the graph of FIG. 29A. According to these results, as described above, even if subjects equally correspond to the moderate risk group of KDIGO grade, when the score of renal disease diagnostic information is high, the renal disease progression rate may be faster than when the score of renal disease diagnostic information is low.

Moreover, as shown in the graph of FIG. 29A, when only considering results according to existing biomarkers, the incidence rate of renal disease-related events of the low-risk group (a1) of KDIGO grade may be higher than the incidence rate of renal disease-related events of the moderate risk group (a2) of KDIGO grade.

However, as shown in the graph of FIG. 29B, the incidence rate of renal disease-related events of Group 2 (b2), which is a group of subjects with scores according to renal disease diagnostic information of 20 or more among the low-risk group of KDIGO grade, may be higher than the incidence rate of renal disease-related events of Group 3 (b3), which is a group of subjects with scores according to renal disease diagnostic information less than 20 among the moderate risk group of KDIGO grade. Accordingly, even if subjects equally have low KDIGO grades, when the score of renal disease diagnostic information is higher than subjects with high KDIGO grades, it may mean that the risk of renal disease is high and the renal disease progression rate may be fast.

Also, the difference in C-statistics when reflecting scores according to renal disease diagnostic information in KDIGO grade compared to using only KDIGO grade is 0.04 (95% confidence interval (CI): 0.02-0.04), and when reflecting scores according to renal disease diagnostic information in KDIGO grade, the risk for renal disease can be predicted with higher accuracy than using only KDIGO grade.

Referring again to FIG. 27, in one embodiment, the processor of the diagnostic device may determine whether the score of renal disease diagnostic information is equal to or greater than a predetermined threshold value.

And, the processor of the diagnostic device may determine that the progression rate of renal disease is fast when the score of renal disease diagnostic information is equal to or greater than the predetermined threshold value, and may determine that the progression rate of renal disease is slow when the score of renal disease diagnostic information is less than the predetermined threshold value.

For example, as in Example 2, even if grades according to KDIGO grade, eGFR level, albuminuria level, and cystatin C level are the same for a first subject and a second subject, when the score of renal disease diagnostic information of the first subject is equal to or greater than a predetermined threshold value (for example, score 20 of renal disease diagnostic information) and the score of renal disease diagnostic information of the second subject is less than the predetermined threshold value, the processor of the diagnostic device may determine that the renal disease progression rate of the first subject is faster than the second subject.

Also, in one embodiment, in step S830, the processor of the diagnostic device may predict Acute kidney injury (AKI) after cardiac surgery. Specifically, after cardiac surgery, kidney injury may or may not occur depending on the patient. For example, after cardiac surgery, 5% of patients suffer from chronic kidney disease, and 1-2% of patients may have completely damaged kidney function and may need to undergo dialysis or kidney transplantation.

The existing biomarkers described above may be used as biomarkers for predicting acute kidney injury after cardiac surgery, but accuracy may not be high. Since the renal disease diagnostic information of the present specification can more accurately predict the risk of kidney injury than existing biomarkers, it can also predict the risk of acute kidney injury after cardiac surgery that existing biomarkers do not predict.

As an example, the processor of the diagnostic device may determine whether the score of renal disease diagnostic information is equal to or greater than a predetermined threshold value. The predetermined threshold value may be the same as or different from the threshold value used for predicting renal disease progression rate. And, when the score of renal disease diagnostic information is equal to or greater than the predetermined threshold value, the processor of the diagnostic device may determine that the possibility of acute kidney injury after cardiac surgery is high, and when the score of renal disease diagnostic information is less than the predetermined threshold value, may determine that the possibility of acute kidney injury after cardiac surgery is low.

And, the processor of the diagnostic device may provide information regarding renal disease progression rate (or information regarding acute kidney injury after cardiac surgery) and/or guide information accordingly. In this case, as the renal disease progression rates (or acute kidney injury possibilities after cardiac surgery) of the first subject and the second subject are different, the processor of the diagnostic device may provide different guide information to the first subject and the second subject.

For example, even when the first subject and the second subject belong to the same risk group according to existing biomarkers and/or renal disease diagnostic information, different guide information may be provided according to the renal disease progression rates of the first subject and the second subject. For example, when the renal disease progression rate of the first subject is predicted to be fast and the first subject is in a low-risk group, the processor of the diagnostic device may provide guide information to the effect that management of blood pressure, diabetes, etc. should be done thoroughly. As an example, the processor of the diagnostic device may provide action information including information regarding additional examinations (for example, explanation of additional examinations, additional examination dates, information regarding hospitals/medical staff where additional examinations are possible, etc.) and/or management information including recommended lifestyle correction goals, recommended dietary information, etc. as guide information.

Also, when the renal disease progression rate of the first subject is predicted to be fast and the first subject is in a high-risk group, the processor of the diagnostic device may increase the dosage of the first subject as prescription information.

Also, the processor of the diagnostic device may perform communication with the monitoring device described above. The processor of the diagnostic device may provide guide information determined according to the renal disease progression rate to the monitoring device. For example, when the processor of the diagnostic device determines that the renal disease progression rate of a first subject in a low-risk group of renal disease diagnostic information who has no symptoms and does not have renal risk factors is fast, action information (for example, additional examination information, etc.) and/or management information (for example, food intake control, target exercise amount, etc.) may be determined as guide information accordingly, and guide information may be provided to the monitoring device. The monitoring device may provide guide information obtained from the diagnostic device to the subject and determine whether the monitored information matches the guide information. As a result of determination, if matched, the monitoring device may provide information that the subject is complying well with the guide information, and if not matched, the monitoring device may provide a warning to the subject to comply with the guide information.

Also, the processor of the diagnostic device may provide guide information based on the possibility of acute kidney injury after cardiac surgery. For example, when it is determined that the possibility of acute kidney injury after cardiac surgery of the subject is high, the processor of the diagnostic device may provide guide information recommended to reduce the possibility of acute kidney injury after cardiac surgery.

Also, the processor of the diagnostic device may provide guide information using a predetermined database and/or guide information model, as described in 2.1.5. In this case, guide information according to renal disease diagnostic information and renal disease progression rate (or information regarding acute kidney injury after cardiac surgery) may be matched and stored in the database, or the guide information model may be learned according to guide information according to renal disease diagnostic information and renal disease progression rate (or acute kidney injury possibility after cardiac surgery). Accordingly, the processor of the diagnostic device may obtain guide information from the database and/or guide information model using renal disease diagnostic information and renal disease progression rate (or acute kidney injury possibility after cardiac surgery).

For example, when in a low-risk group in terms of eGFR level and the score of renal disease diagnostic information is equal to or greater than a predetermined threshold value, the processor of the diagnostic device may determine that the renal risk of the subject is low, but the renal disease progression rate of the subject is faster than other low-risk groups. Accordingly, the processor of the diagnostic device may provide information that the renal disease progression rate is fast and/or guide information accordingly (for example, guide information provided when renal disease diagnostic information is determined to be moderate risk).

Also, when in a low-risk group in terms of eGFR level and the score of renal disease diagnostic information is less than a predetermined threshold value, the processor of the diagnostic device may determine that the renal risk of the subject is also low and the renal disease progression rate of the subject is slow compared to other low-risk groups. Accordingly, the processor of the diagnostic device may provide information that the renal disease progression rate is slow and/or guide information accordingly (for example, guide information provided when renal disease diagnostic information is determined to be low risk).

Also, according to embodiments, different threshold values may be applied to renal disease diagnostic information according to result values according to biomarkers. For example, the threshold value when the KDIGO grade is a low-risk group, the threshold value when it is a moderate risk group, and the threshold value when it is a high-risk group may be set differently in the diagnostic device.

Also, according to embodiments, the threshold value may be set based on various criteria. For example, the threshold value may be set so that the incidence rate of renal disease occurrence events is clearly stratified when renal disease diagnostic information is applied to result values according to biomarkers.

Various embodiments of the present specification may be implemented as software including instructions stored in a machine-readable storage media that can be read by a machine (eg: computer). The machine is a device capable of calling stored instructions from a storage medium and operating according to the called instructions, and may include electronic devices according to the disclosed embodiments. When the instruction is executed by a processor, the processor may perform functions corresponding to the instruction directly or using other components under the control of the processor. The instruction may include code generated or executed by a compiler or interpreter. The machine-readable storage medium may be provided in the form of a non-transitory storage medium. Here, 'non-transitory storage medium' means that it does not include signals and is tangible, and does not distinguish whether data is stored semi-permanently or temporarily in the storage medium. For example, a 'non-transitory storage medium' may include a buffer in which data is temporarily stored.

According to one embodiment, methods according to various embodiments disclosed in the present specification may be provided by being included in a computer program product. The computer program product may be traded between sellers and buyers as a product. The computer program product may be distributed in the form of a machine-readable storage medium (eg: Compact Disc Read Only Memory, CD-ROM) or online through an application store (eg: Play Store™). In the case of online distribution, at least part of the computer program product (eg: downloadable app) may be at least temporarily stored or temporarily generated in a storage medium such as a memory of a manufacturer's server, an application store's server, or a relay server.

As described above, although embodiments have been described with limited embodiments and drawings, those having ordinary knowledge in the technical field can make various modifications and variations from the above description. For example, even if the described technologies are performed in a different order from the described method, and/or components of the described system, structure, device, circuit, etc. are combined or combined in a different form from the described method, or are replaced or substituted by other components or equivalents, appropriate results may be achieved.

Therefore, other implementations, other embodiments, and equivalents to the claims also belong to the scope of the claims described below.

**Claims**

1. A control method of a diagnostic device, the method comprising:

    obtaining a retinal image of a subject; and
    obtaining renal disease diagnostic information regarding the subject using a machine learning model based on the retinal image,
    wherein the machine learning model includes a first model and a second model,
    wherein the first model is a neural network model, and
    wherein the second model is a regression-based machine learning model.

2. The control method of a diagnostic device of claim 1,

    wherein the first model is trained based on first training data including a plurality of retinal images and a result value according to a first biomarker corresponding to the first training data, and
    wherein the obtaining of renal disease diagnostic information regarding the subject comprises:

        inputting the retinal image to the first model to obtain a first result value from the first model;
        inputting the first score and physical information of the subject to the second model to obtain a second result value from the second model; and
        obtaining the renal disease diagnostic information based on the second result value.

3. The control method of a diagnostic device of claim 2,

    wherein the result value according to the first biomarker is at least one of a probability that an Estimated Glomerular Filtration Rate (eGFR) is equal to or less than a first numerical value or information on whether albuminuria is present in the urine of the subject, and
    wherein the first result value indicates at least one of the probability that the eGFR of the subject is equal to or less than the first numerical value or the probability that albuminuria is present in the urine of the subject.

4. The control method of a diagnostic device of claim 3,

wherein the second machine learning model is trained based on second training data, and
wherein the second training data includes a follow-up result of renal disease events of subjects in a predetermined population.

5. The control method of a diagnostic device of claim 4,
wherein data regarding subjects in the predetermined population whose eGFR is less than a second numerical value is excluded from the second training data, wherein the second numerical value being greater than the first numerical value.

6. The control method of a diagnostic device of claim 1,

wherein the first result value includes information regarding the current risk of renal disease of the subject, and
wherein the second result value includes information regarding the probability of future renal disease events for the subject.

7. The control method of a diagnostic device of claim 6,
wherein the second result value includes information regarding the probability of renal disease events within 5 years for the subject.

8. The control method of a diagnostic device of claim 1,
wherein the obtaining of renal disease diagnostic information regarding the subject comprises applying a predetermined cut-off value to the second result value to obtain, as the renal disease diagnostic information, any one grade corresponding to the subject among a plurality of grades.

9. The control method of a diagnostic device of claim 8,
wherein the cut-off value is set based on population distribution in groups according to a plurality of grades categorized based on a follow-up observation result of a predetermined population.

10. The control method of a diagnostic device of claim 1, further comprising providing guide information to the subject based on the renal disease diagnostic information.

11. The control method of a diagnostic device of claim 10,
wherein the providing of guide information to the subject based on the renal disease diagnostic information comprises providing guide information corresponding to the renal disease diagnostic information using a pre-stored database or a machine learning model.

12. The control method of a diagnostic device of claim 10, further comprising determining a progression rate of the renal disease of the subject based on the renal disease diagnostic information,
wherein the determining of the progression rate of the renal disease comprises obtaining a result value according to a biomarker of the subject and determining the progression rate of the renal disease of the subject using the result value according to the biomarker and the second result value.

13. The control method of a diagnostic device of claim 12,

wherein if the second result value is equal to or greater than a predetermined value, the renal disease is determined to be progressing faster than the renal disease progression rate expected based on the result value according to the biomarker; and
wherein if the second result value is less than the predetermined value, the renal disease is determined to be progressing slower than the renal disease progression rate expected based on the result value according to the biomarker.

14. The control method of a diagnostic device of claim 12,
wherein the providing of guide information to the subject based on the renal disease diagnostic information comprises providing guide information to the subject using the progression rate of the renal disease of the subject and the renal disease diagnostic information.

15. A recording medium having a program recorded thereon for performing the method of any one of claims 1 to 14.

16. A diagnostic device, comprising:

a storage module; and
at least one processor;
wherein the at least one processor is configured to:

obtain a retinal image of a subject; and
obtain renal disease diagnostic information regarding the subject using a machine learning model stored in the storage module based on the retinal image; wherein the machine learning model includes a first model and a second model; wherein the first model is a neural network model; and wherein the second model is a regression-based machine learning model.

## FIG.1

## FIG.2

20

| STORAGE MODULE | | PROCESSOR | | COMMUNICATION MODULE |
|---|---|---|---|---|
| 21 | ↔ | 22 | ↔ | 23 |

# FIG.3

2

40

DIAGNOSTIC SERVER

| FIRST LEARNING DEVICE | SECOND LEARNING DEVICE | FIRST CLIENT DEVICE | SECOND CLIENT DEVICE |
|---|---|---|---|
| 10a | 10b | 30a | 30b |

# FIG.4

<u>30</u>

31                          32                          33

```
IMAGING          PROCESSOR        COMMUNICATION
MODULE                              MODULE
```

# FIG.5

### LEARNING PROCESS

S11                    S12                    S13

DATA SET → DATA PROCESSING → DIAGNOSTIC MODEL LEARNING → MODEL PARAMETER ACQUISITION

### DIAGNOSTIC PROCESS

S21                    S22                    S23

DIAGNOSTIC TARGET DATA → DATA ACQUISITION → LEARNED DIAGNOSTIC MODEL → DIAGNOSTIC INFORMATION ACQUISITION → DIAGNOSTIC INFORMATION

# FIG.6

100

LEARNING UNIT

| 110 | 130 | 150 | 170 |
|---|---|---|---|
| DATA PROCESSING MODULE | QUEUE MODULE | LEARNING MODULE | LEARNING RESULT ACQUISITION MODULE |

# FIG.7

DS

IMAGE DATA SET

FIRST IMAGE DATA — ID1

FIRST IMAGE — I1

FIRST LABEL — L1

SECOND IMAGE DATA — ID2

nTH IMAGE DATA — IDn

# FIG.8

LEARNING PROCESS

S31

DATA
SET → DATA
ACQUISITION → S32

DIAGNOSTIC
MODEL → S33

MODEL
VERIFICATION → S34

PARAMETER
ACQUISITION → PARAMETER

# FIG.9

200

DIAGNOSTIC UNIT

DIAGNOSTIC
TARGET
DATA → 210

DIAGNOSTIC
REQUEST
ACQUISITION
MODULE → 230

DATA
PROCESSING
MODULE → 250

DIAGNOSTIC
MODULE → 270

OUTPUT
MODULE → DIAGNOSTIC
INFORMATION

# FIG.10

DIAGNOSTIC PROCESS

DIAGNOSTIC TARGET DATA → | S41 DATA ACQUISITION | → | S42 DIAGNOSTIC MODEL | → | S43 RESULT OUTPUT | → DIAGNOSTIC INFORMATION

# FIG.11

200

DIAGNOSTIC UNIT

DIAGNOSTIC TARGET DATA → | 211 DIAGNOSTIC REQUEST ACQUISITION MODULE | → | 231 DATA PROCESSING MODULE | → | 251 FIRST DIAGNOSTIC MODULE | → | 271 OUTPUT MODULE | → DIAGNOSTIC INFORMATION

253 SECOND DIAGNOSTIC MODULE

# FIG.12

DIAGNOSTIC PROCESS

S52a

FIRST
DIAGNOSTIC
MODEL

S51

DIAGNOSTIC
TARGET DATA

DATA
ACQUISITION

S53

RESULT
OUTPUT

DIAGNOSTIC
INFORMATION

SECOND
DIAGNOSTIC
MODEL

S52b

## FIG.13

20

DIAGNOSTIC UNIT

25

FIRST
PROCESSING
UNIT

24

FUNDUS
IMAGE

FUNDUS
IMAGE
ACQUISITION
UNIT

27

THIRD
PROCESSING
UNIT

28

DIAGNOSTIC
INFORMATION
OUTPUT UNIT

DIAGNOSTIC
INFORMATION

SECOND
PROCESSING
UNIT

26

## FIG.14

1000

DIAGNOSTIC MODEL

1100                1200

INPUT → FIRST DIAGNOSTIC MODEL → SECOND DIAGNOSTIC MODEL → DIAGNOSTIC INFORMATION

FIG.15

1000

DIAGNOSTIC MODEL

1100                1200

FIRST INPUT → FIRST DIAGNOSTIC MODEL → SECOND DIAGNOSTIC MODEL → DIAGNOSTIC INFORMATION

SECOND INPUT

FIG.16

1000

DIAGNOSTIC MODEL

| INPUT | → | 1100<br>FIRST<br>DIAGNOSTIC<br>MODEL | → | 1200<br>SECOND<br>DIAGNOSTIC<br>MODEL | → | SECOND<br>DIAGNOSTIC<br>INFORMATION |

FIRST
DIAGNOSTIC
INFORMATION

# FIG.17

START

OBTAIN INPUT DATA — S61

OBTAIN FIRST DIAGNOSTIC INFORMATION — S62

OBTAIN SECOND DIAGNOSTIC INFORMATION — S63

END

# FIG.18

START

OBTAIN RETINAL IMAGE ~S100

OBTAIN RENAL DISEASE DIAGNOSTIC
ASSISTANT INFORMATION ~S200

END

# FIG.19

1000

DIAGNOSTIC MODEL

1100

1200

INPUT

FIRST
DIAGNOSTIC
MODEL

SECOND
DIAGNOSTIC
MODEL

RENAL DISEASE
DIAGNOSTIC
INFORMATION

# FIG.20

| | KOREAN HEALTH SCREENING CENTER (n=79,108) | UK BIOBANK (n=30,477) | KOREAN DIABETIC COHORT (n=5,014) |
|---|---|---|---|
| AGE, MEAN(SD), YEARS | 49.5 ±11.8 | 54.2 ± 8.1 | 55.1 ± 10.5 |
| MALE, No(%) | 44,364 (56.1) | 13,617 (44.7) | 2,814 (56.1) |
| DIABETES, No(%) | 5,349 (6.7) | 1,293 (4.2) | 5,014 (100) |
| HYPERTENSION, No(%) | NA | 4,050 (13.3) | 1,400 (27.9) |
| eGFR, mL/min/1.73 m$^2$ | 100.3 ± 14.3 | 99.4 ± 6.6 | 102.5 ± 9.1 |

FIG.21

FIG.22A

FIG.22B

| | eGFR-BASED RENAL DISEASE SCORE | SCORE ACCORDING TO RENAL DISEASE DIAGNOSTIC INFORMATION | $p$ value |
|---|---|---|---|
| **UK BIOBANK** | | | |
| C-STATISTICS | 0.618 (0.598–0.638) | 0.638 (0.618–0.658) | |
| Δ C-STATISTICS | Ref | 0.020 (0.011–0.029) | <0.001 |
| NRI | | 0.109 (0.044–0.156) | <0.001 |
| **KOREAN DIABETIC COHORT** | | | |
| C-STATISTICS | 0.679 (0.642–0.717) | 0.703 (0.664–0.742) | |
| Δ C-STATISTICS | Ref | 0.024 (0.002–0.046) | 0.002 |
| NRI | | 0.179 (0.017–0.292) | 0.03 |

FIG.23

START

OBTAIN RETINAL IMAGE ~ S300

OBTAIN INFORMATION REGARDING RENAL DISEASE RISK
ACCORDING TO EXISTING BIOMARKERS AS RENAL
DISEASE DIAGNOSTIC ASSISTANT INFORMATION ~ S400

END

# FIG.24

START

OBTAIN RENAL DISEASE DIAGNOSTIC
ASSISTANT INFORMATION ~ S500

PROVIDE GUIDE INFORMATION ~ S600

END

# FIG.25

START

OBTAIN EXISTING PRESCRIPTION INFORMATION OF SUBJECT ⟼ S710

OBTAIN RENAL DISEASE DIAGNOSTIC ASSISTANT INFORMATION ⟼ S720

PROVIDE GUIDE INFORMATION BASED ON THE EXISTING PRESCRIPTION INFORMATION AND RENAL DISEASE DIAGNOSTIC INFORMATION ⟼ S730

END

# FIG.26

START

OBTAIN RESULT VALUE ACCORDING TO BIOMARKER ⟼ S810

OBTAIN RENAL DISEASE DIAGNOSTIC ASSISTANT INFORMATION ⟼ S820

DETERMINE PROGRESSION RATE OF RENAL DISEASE USING RESULT VALUE ACCORDING TO BIOMARKER AND RENAL DISEASE DIAGNOSTIC INFORMATION ⟼ S830

END

# FIG.27

| | TOTAL | KDIGO GRADE | | |
|---|---|---|---|---|
| | | LOW-RISK GROUP | MODERATE RISK GROUP | HIGH-RISK GROUP |
| n | N=5,346 | N=3,135 | N=1,814 | N=397 |
| AGE, year | 62.4 ± 11.4 | 64.5 ± 10.1 | 59.2 ± 12.3 | 60.5 ± 12.5 |
| MALE, n | 3,241 (60.6) | 1,903 (60.7) | 1,080 (59.5) | 258 (65.0) |
| GLYCATED HEMOGLOBIN, % | 7.4 ± 1.5 | 7.1 ± 1.2 | 7.8 ± 1.7 | 8.0 ± 1.8 |
| eGFR, ml/min/1.73 $m^2$ | 86.6 ± 15.2 | 83.2 ± 10.8 | 93.8 ± 16.4 | 80.8 ± 24.6 |
| HYPERTENSION | 2,244 (42.0) | 1,260 (40.2) | 778 (42.9) | 206 (51.9) |
| ALBUMINURIA GRADE(mg/g) | | | | |
| A1 (<30) | 2,454 (53.2) | 2,406 (100.0) | 34 (1.9) | 14 (3.5) |
| A2 (30-300) | 1,818 (39.4) | 0 | 1,780 (98.1) | 38 (9.6) |
| A3 (>300) | 345 (7.5) | 0 | 0 | 345 (86.9) |
| eGFR GRADE (ml/min/1.73 $m^2$) | | | | |
| G1 (≥90) | 1,730 (32.4) | 457 (14.6) | 1,119 (61.7) | 154 (38.8) |
| G2 (60-89) | 3,500 (65.5) | 2,678 (85.4) | 661 (36.4) | 161 (40.6) |
| G3a (45-59) | 70 (1.3) | 0 | 34 (1.9) | 36 (9.1) |
| G3b (30-44) | 37 (0.7) | 0 | 0 | 37 (9.3) |
| G4 (15-29) | 9 (0.2) | 0 | 0 | 9 (2.3) |

FIG.28

FIG.29A

RENAL DISEASE RISK GROUP

——————— LOW-RISK GROUP WITH RENAL DISEASE DIAGNOSTIC
INFORMATION SCORE LESS THAN 20 (b1)

– – – LOW-RISK GROUP WITH RENAL DISEASE DIAGNOSTIC
INFORMATION SCORE 20 OR MORE (b2)

——— MODERATE RISK GROUP WITH RENAL DISEASE DIAGNOSTIC
INFORMATION SCORE LESS THAN 20 (b3)

– – – MODERATE RISK GROUP WITH RENAL DISEASE DIAGNOSTIC
INFORMATION SCORE 20 OR MORE (b4)

·········· HIGH-RISK GROUP (b5)

INCIDENCE RATE OF CHRONIC KIDNEY
DISEASE-RELATED EVENTS (%)

TIME (YEARS)

# FIG.29B

## INTERNATIONAL SEARCH REPORT

| International application No. |
| --- |
| **PCT/KR2023/017322** |

| A. | CLASSIFICATION OF SUBJECT MATTER |
| --- | --- |

**A61B 3/00**(2006.01)i; **A61B 3/12**(2006.01)i; **A61B 3/14**(2006.01)i; **G16H 50/20**(2018.01)i; **G16H 30/20**(2018.01)i

According to International Patent Classification (IPC) or to both national classification and IPC

| B. | FIELDS SEARCHED |
| --- | --- |

Minimum documentation searched (classification system followed by classification symbols)

A61B 3/00(2006.01); A61B 5/00(2006.01); G06N 3/02(2006.01); G06N 3/04(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 망막(retina), 이미지(image), 신장(kidney), 진단(diagnosis), 신경망(neural network), 머신러닝(machine learning)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2021-0146690 A (SPIDERCORE INC.) 06 December 2021 (2021-12-06) <br> See claims 1-9. | 1-16 |
| Y | KR 10-2019-0115713 A (KOREA UNIVERSITY RESEARCH AND BUSINESS FOUNDATION) 14 October 2019 (2019-10-14) <br> See paragraphs [0062] and [0063]. | 1-16 |
| A | KR 10-2058883 B1 (HONGBOG) 24 December 2019 (2019-12-24) <br> See claim 1. | 1-16 |
| A | JP 2011-212094 A (FUJIFILM CORP.) 27 October 2011 (2011-10-27) <br> See entire document. | 1-16 |
| A | KR 10-2022-0122573 A (MEDIWHALE INC.) 02 September 2022 (2022-09-02) <br> See entire document. | 1-16 |

| ✓ Further documents are listed in the continuation of Box C. | ✓ See patent family annex. |
| --- | --- |

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **06 March 2024** | **07 March 2024** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 725 391 A1**

**INTERNATIONAL SEARCH REPORT**

| International application No. |
| --- |
| **PCT/KR2023/017322** |

**C.    DOCUMENTS CONSIDERED TO BE RELEVANT**

| Category* | Citation of document, with indication, where appropriate, of the relevant passages | Relevant to claim No. |
| --- | --- | --- |
| X | JOO, Young Su et al. Retinal Photograph-Based Deep Learning Predicts CKD Among People With Preserved Kidney Function. 04 November 2022. [Retrieved on 15 February 2024]. Retrieved from <KIDNEY WEEK. URL: https://www.asn-online.org/education/kidneyweek/2022/program-abstract.aspx?controlId=3766433>.<br><br>See pages 1-3.<br><br>※ This document is a known document declaring exceptions to lack of novelty by the applicant. | 1-16 |

Form PCT/ISA/210 (second sheet) (July 2022)

**INTERNATIONAL SEARCH REPORT**
Information on patent family members

| International application No. |
| PCT/KR2023/017322 |

| Patent document cited in search report | | | Publication date (day/month/year) | Patent family member(s) | | | Publication date (day/month/year) |
|---|---|---|---|---|---|---|---|
| KR | 10-2021-0146690 | A | 06 December 2021 | KR | 10-2410292 | B1 | 17 June 2022 |
| | | | | WO | 2021-241830 | A1 | 02 December 2021 |
| KR | 10-2019-0115713 | A | 14 October 2019 | KR | 10-2143940 | B1 | 13 August 2020 |
| KR | 10-2058883 | B1 | 24 December 2019 | | None | | |
| JP | 2011-212094 | A | 27 October 2011 | JP | 5320335 | B2 | 23 October 2013 |
| KR | 10-2022-0122573 | A | 02 September 2022 | KR | 10-2022-0001162 | A | 05 January 2022 |
| | | | | KR | 10-2023-0152647 | A | 03 November 2023 |
| | | | | KR | 10-2435799 | B1 | 25 August 2022 |
| | | | | KR | 10-2596534 | B1 | 01 November 2023 |
| | | | | US | 2023-0162359 | A1 | 25 May 2023 |
| | | | | WO | 2022-004956 | A1 | 06 January 2022 |

Form PCT/ISA/210 (patent family annex) (July 2022)